# EUROPÄISCHE PATENTSCHRIFT

(11) **EP 1 912 735 B1**
(45) Veröffentlichungstag und Bekanntmachung des Hinweises auf die Patenterteilung: **15.04.2009**
(21) Anmeldenummer: 06778120.3
(22) Anmeldetag: 02.08.2006
(51) Int. Cl.: B01J 23/00, B01J 37/00, C07C 253/26, C07C 255/08, C07C 51/25, C07C 57/04

(54) **VERFAHREN ZUR HERSTELLUNG VON KATALYSATORFORMKÖRPERN, DEREN AKTIVMASSE EIN MISCHOXID IST**
PROCESS FOR PRODUCING CATALYST BODIES WHOSE ACTIVE MASS IS A MIXED OXIDE
PROCEDE DE PREPARATIO DE CORPS CATALYTIQUES DONT LA MASSE ACTIVE EST UN OXYDE MIXTE

(30) Priorität: 05.08.2005 DE 102005037678; 05.08.2005 US 705485 P
(43) Veröffentlichungstag der Anmeldung: 23.04.2008
(73) Patentinhaber: BASF SE, 67056 Ludwigshafen (DE)
(72) Erfinder: HIBST, Hartmut, 69198 Schriesheim (DE); FELDER, Raimund, 67434 Neustadt (DE); ARNOLD, Heiko, Zijin Villas, Nanjing 210014 (CN); RAICHLE, Andreas, 67063 Ludwigshafen (DE); MÜLLER-ENGEL, Klaus Joachim, 76297 Stutensee (DE)
(86) Internationale Anmeldenummer: PCT/EP2006/064948
(87) Internationale Veröffentlichungsnummer: WO 2007/017431

(56) Entgegenhaltungen:
- EP-A2- 0 184 790
- WO-A-20/05030393
- WO-A2-02/062737
- DATABASE WPI Week 197625 Derwent Publications Ltd., London, GB; AN 1976-46673X XP002410647 & JP 49 130888 A (DENKI KAGAKU KOGYO KK) 14. Dezember 1974 (1974-12-14)

## Beschreibung

Vorliegende Erfindung betrifft ein Verfahren zur Herstellung von Katalysatorformkörpern, deren Aktivmasse ein Multielementoxid ist, bei dem man ein feinteiliges Vorläufergemisch, das ein feinteiliges Formungshilfsmittel zugesetzt enthält, zur gewünschten Geometrie formt (verdichtet) und die dabei resultierenden Katalysatorvorläuferformkörper bei erhöhter Temperatur unter Erhalt von Katalysatorformkörpern, deren Aktivmasse ein Multielementoxid ist, thermisch behandelt.

Ferner betrifft vorliegende Erfindung ein Verfahren von heterogen katalysierten Gasphasenreaktionen, z.B. Gasphasenpartialoxidation organischer Verbindungen, bei denen vorgenannte Katalysatorformkörper als Katalysatoren verwendet werden.

Beispiele für solche heterogen katalysierten Partialoxidationsverfahren sind die Herstellung von Acrolein aus Propylen sowie die Herstellung von Methacrylsäure aus Methacrolein, wie sie z.B. in der WO 2005/030393 und in der EP-A 467 144 beschrieben sind. Partialoxidationsprodukte organischer Verbindungen sind wichtige Zwischenprodukte. Acrolein bildet beispielsweise ein wichtiges Zwischenprodukt bei der Herstellung von Acrylsäure, die durch heterogen katalysierte partielle Oxidation von Acrolein erhältlich ist. Acrylsäure bildet ein bedeutendes Monomeres, das als solches oder in Form seiner Alkylester radikalisch polymerisiert werden kann. Die resultierenden Polymerisate eignen sich u.a. als superabsorbierende Materialien oder als Klebstoffe. In entsprechender Weise eignet sich auch Methacrylsäure als solche oder in Form ihrer Alkylester zur Herstellung radikalischer Polymerisate. Eine herausragende Position nimmt dabei z.B. der Methylester der Methacrylsäure ein, der insbesondere zur Herstellung von Polymethylmethacrylat Verwendung findet, das als Kunststoffglas eingesetzt wird.

Eingangs beschriebene Verfahren zur Herstellung von Katalysatorformkörpern, deren Aktivmasse ein Multimetalloxid ist, sind beispielsweise in der US 2005/0131253 A1 bekannt. Der Zusatz eines Formungshilfsmittels (z.B. Gleitmittels) bei der Katalysatorformkörperherstellung erfolgt dabei u.a. um die mechanische Abnutzung der Formungswerkzeuge zu mindern. Üblicherweise wird als Formungshilfsmittel (z.B. Gleitmittel) feinteiliger Graphit verwendet. Seine Mitverwendung wirkt sich aber auch vorteilhaft auf das innere Gefüge und den inneren Zusammenhalt sowohl der Katalysatorvorläuferformkörper als auch der resultierenden Katalysatorformkörper aus. Da Graphit als Kohlenstoff enthaltende Substanz brennbar ist, erfolgt die thermische Behandlung der das feinteilige Graphit zugesetzt enthaltenden Katalysatorvorläuferformkörper in an sich bekannter Weise in der Regel so, dass sich das Graphit während der thermischen Behandlung nicht entzündet. Dies erfordert insbesondere dann eine gewisse Sorgfalt, wenn die Atmosphäre in welcher die thermische Behandlung erfolgt, molekularen Sauerstoff enthält (die oxidierende Wirkung kann aber auch aus der Vorläufermasse selbst heraus resultieren). Da natürlich vorkommender Graphit ein Gemisch aus Graphit und mineralischen Bestandteilen ist, die die Entzündung des Graphits zu katalysieren vermögen und die Katalysatorperformance gegebenenfalls beeinträchtigen, wird normalerweise synthetischer Graphit verwendet, dessen Zündtemperatur im wesentlichen nur von seiner Körnung abhängt. Bei Verwendung natürlicher Graphite ist bei gegebener Körnung und spezifischer Oberfläche die Zündtemperatur in an sich bekannter Weise davon abhängig, von welcher Art und Menge ihre mineralischen Gehalte sind. Der Wechsel zu einer neuen Quelle natürlichen Graphits ginge daher in der Regel mit aufwendigen Verfahren der Anpassung des Herstellverfahrens einher. Dies ist ein weiterer Nachteil einer Verwendung von natürlichem Graphit als Gleitmittel bei der relevanten Herstellung von Katalysatorformkörpern. Verbrennt der Graphit während der Herstellung derselben, so werden normalerweise diejenigen Temperaturen, die für die thermische Behandlung der Katalysatorvorläuferformkörper ins Auge gefasst wurden, signifikant überschritten, was im Regelfall eine Minderung der Katalysatorperformance im Rahmen der heterogen zu katalysierenden Gasphasenreaktion bedingt. Ein weiterer Nachteil einer Verwendung von Graphit als Formungshilfsmittel liegt darin begründet, dass der im Katalysatorformkörper verbleibende Graphit während der Laufzeit des Katalysators im Rahmen seiner Nutzung für eine heterogen katalysierte Gasphasenreaktion, insbesondere einer partielle Oxidation, das Multimetalloxid reduktiv beeinträchtigt.

Vorteilhaft an einer Verwendung von feinteiligem Graphit als Formungshilfsmittel, z.B. als Gleitmittel, ist jedoch, dass das im Rahmen der thermischen Behandlung im Katalysatorformkörper verbleibende Graphit sich bezüglich der meisten heterogen katalysierten Gasphasenreaktionen normalerweise inert verhält, d.h., keine Störung bewirkt. Typische Graphiteinsatzmengen belaufen sich auf 0,1 bis 20 bzw. bis 10 Gew.-%, bezogen auf die Masse des Katalysatorvorläuferformkörpers.

Die Aufgabe der vorliegenden Erfindung bestand daher darin, ein verbessertes Verfahren zur Herstellung von Katalysatorformkörpern unter Mitverwendung eines Formungshilfsmittels zur Verfügung zu stellen, das einerseits die Nachteile einer Verwendung von Graphit nicht mehr bzw. in geminderter Form aufweist, die Vorteile einer solchen aber beibehält.

Demgemäss wurde ein Verfahren zur Herstellung von Katalysatorformkörpern, deren Aktivmasse ein Multielementoxid ist und bei dem man ein feinteiliges Vorläufergemisch, das ein feinteiliges Formungshilfsmittel zugesetzt enthält, zur gewünschten Geometrie formt (verdichtet) und die dabei resultierenden Katalysatorvorläuferformkörper bei erhöhter Temperatur unter Erhalt der Katalysatorformkörper, deren Aktivmasse ein Multielementoxid ist, thermisch behandelt, gefunden, das dadurch gekennzeichnet ist, dass das feinteilige Vorläufergemisch als Formungshilfsmittel Bornitrid zugesetzt enthält.

Erfindungsgemäß bevorzugt enthält das feinteilige Vorläufergemisch das Bornitrid in feinteiliger Form zugesetzt. Besonders vorteilhafte Bornitride (BN) sind für das erfindungsgemäße Verfahren die feinteiligen Bornitride der Firma H.C. Starck, P.O. Box 2540, 38615 Goslar/Germany. Unter diesen sind vor allem die nachfolgend genannten Bornitride für den erfindungsgemäßen Verwendungszweck günstig:
a) Boron Nitride Grade A 01 (Number PD-5006, Issue 0-07.99) Hierbei handelt es sich um weißes, hexagonales Pulver. Die zugehörige HS Number lautet: 28500030. Die spezifische BET-Oberfläche beträgt 4,0 bis 7,5 m²/g (auch in allen nachfolgenden Fällen Areameter II by BET per DIN 66132).
Die Tap Density liegt bei 0,2 bis 0,5 g/cm³ (auch in allen nachfolgenden Fällen gemäß ASTM B 527 (25ml grad. Cylinder)). Das Pulver weist einen hohen Kristallisationsgrad auf. Die Gehalte des Bornitridpulvers sind wie folgt (auch in allen nachfolgenden Fällen Gew.-% bezogen auf die Gesamtmasse):

| | |
|---|---|
| B: | 42,5 bis 43,5 Gew.-%, |
| O: | 0,5 bis 1,2 Gew.-%, |
| B₂O₃ (wasserlöslich): | ≤ 0,15 Gew.-%, |
| H₂O: | ≤ 0,15 Gew.-% und |
| C: | ≤ 0,10 Gew.-%. |

b) Boron Nitride Grade B50 (Number PD-5006, Issue 0-07.99) Hierbei handelt es sich um weißes, hexagonales Pulver. Die zugehörige HS Number latuet: 28500030. Die spezifische BET-Oberfläche beträgt 4,0 bis 6,5 m²/g. Die Tap Density liegt bei 0,2 bis 0,5 g/cm³. Das Pulver weist einen hohen Kristallisationsgrad auf. Die Gehalte des Bornitridpulvers sind wie folgt:

| | |
|---|---|
| B: | 41,5 bis 43,5 Gew.-%, |
| O: | ≤ 6,0 Gew.-% |
| B₂O₃ (wasserlöslich): | ≤ 5,0 Gew.-%, |
| H₂O: | ≤ 0,7 Gew.-% und |
| C: | ≤ 0,2 Gew-%. |

c) Boron Nitride Grade T15 (Number PD-5180, Issue 0-22.04.2003) Hierbei handelt es sich um weißes, hexagonales Pulver. Die zugehörige HS Number lautet: 28500020. Die spezifische BET-Oberfläche beträgt 10 bis 20 m²/g. Die Tap Density liegt bei 0,2 bis 0,5 g/cm³. Das Pulver weist einen hohen Kristallisationsgrad auf. Die Gehalte des Bornitridpulvers sind wie folgt:

| | |
|---|---|
| B: | 42,5 bis 43,5 Gew.-%, |
| O: | ≤ 1,0 Gew.-%, |
| B₂O₃ (wasserlöslich): | ≤ 0,3 Gew.-%, |
| H₂O: | ≤ 0,15 Gew.-% und |
| C: | ≤ 0,10 Gew.-%. |

d) Boron Nitride Grade B100 (Number PD-5002, Issue 0-07.99) Hierbei handelt es sich um hexagonales, agglomeriertes, gräuliches Pulver. Die zugehörige HS Number lautet: 28500030. Die Tap Density beträgt 0,3 bis 0,8 g/cm³. Die Gehalte des Bornitridpulvers sind wie folgt:

| | |
|---|---|
| B: | 37,0 bis 43,5 Gew.-%, |
| B₂O₃ (wasserlöslich): | ≤ 7,5 Gew.-%, |
| H₂O: | ≤ 1,0 Gew.-% und |
| C: | ≤ 6,0 Gew.-%. |

e) Boron Nitride Grade C (Number PD-5004, Issue 1-01.06.2001) Hierbei handelt es sich um hexogonales, agglomeriertes, weißes Pulver. Die zugehörige HS Number lautet: 28500030. Die spezifische BET-Oberfläche beträgt 10 bis 20 m²/g. Die Tap Density liegt bei 0,25 bis 0,5 g/cm³. Die Gehalte des Bornitridpulvers sind wie folgt:

| | |
|---|---|
| B: | ≥ 41,0 Gew.-%, |
| O: | ≤ 7,0 Gew.-%, |
| B₂O₃ (wasserlöslich): | 5,0 bis 8,0 Gew.-%, |
| H₂O: | ≤ 0,7 Gew.-% und |
| C: | ≤ 0,1 Gew.-%. |

Generell sind erfindungsgemäß Bornitride bevorzugt, die neben den genannten Bestandteilen keine sonstigen Bestandteile enthalten.

Die vorgenannten Bornitridpulver eignen sich insbesondere für alle erfindungsgemäßen Herstellungen von Katalysatorformkörpern, deren Aktivmasse ein Multielementoxid ist, und auf die in dieser Schrift Bezug genommen wird. Dies trifft insbesondere auf die beispielhaft ausgeführten Katalysatorformkörper zu.

Für das erfindungsgemäße Verfahren besonders vorteilhaft ist die Verwendung von Bornitriden, deren Gehalt an wasserlöslichem B₂O₃ ≤ 5 Gew.-%, vorzugsweise ≤ 3 Gew.-% und besonders bevorzugt ≤ 1 Gew.-% bzw. am besten 0 Gew.-% beträgt. Dies wirkt sich günstig auf seine Oxidationsresistenz aus. Häufig liegt der Gehalt an wasserlöslichem B₂O₃ in gleicher Weise bezogen bei ≥ 0,05 Gew.-%.

Desweiteren ist es für das erfindungsgemäße Verfahren günstig, wenn das Bornitrid zu wenigstens 50 Gew.-%, bevorzugt zu wenigstens 75 Gew.-% und ganz besonders bevorzugt ausschließlich in der hexagonalen Phase vorliegt und eine hohe Kristallinität aufweist.

Ganz besonders günstig ist für das erfindungsgemäße Verfahren (insbesondere für die Herstellung aller Katalysatorformkörper auf die in dieser Schrift Bezug genommen wird, vor allem alle beispielhaft ausgeführten) die Verwendung des Boron Nitride Grade A 01, Number PD-5006, Issue 0-07.99, das vorstehend unter a) ausgeführt worden ist.

Erfindungsgemäß zweckmäßig bewegt sich der Partikeldurchmesser des für das erfindungsgemäße Verfahren zu verwendenden feinteiligen Bornitrid im Bereich 1 µm bis 50 µm, vorzugsweise im Bereich 1 bis 10 µm bzw. bis 5 µm (Elektronenmikroskop bzw. Elektronentransmissionsmikroskop). Üblicherweise bewegen sich jeweils wenigstens 50% (jeweils bezogen auf die Gesamtzahl der Partikel), vorzugsweise wenigstens 70% und besonders bevorzugt wenigstens 90% der Partikeldurchmesser in den vorgenannten Bereichen. In der Regel weisen die Partikel eine blättchenförmige Gestalt auf. Der Partikeldurchmesser meint in dieser Schrift stets die längste direkte Verbindung zweier auf der Partikeloberfläche befindlicher Punkte.

In der Regel enthält das feinteilige Vorläufergemisch beim erfindungsgemäßen Verfahren, bezogen auf sein Gesamtgewicht, insgesamt 0,1 bis 10 Gew.-%, bzw. gegebenenfalls bis 20 Gew.-%, häufig 0,3 bis 8 Gew.-%, vielfach 0,5 bis 6 Gew.-%, bzw. 0,5 bis 5 Gew.-% an feinteiligem Formungshilfsmittel zugesetzt. Im Normalfall wird Bornitrid das einzige dem feinteiligen Vorläufergemisch zugesetzte Formungshilfsmittel (z.B. Gleitmittel) sein. D.h., bezogen auf sein Gesamtgewicht (einschließlich des zugesetzten Bornitrids) wird man dem feinteiligen Vorläufergemisch in diesen Fällen 0,1 bis 10 Gew.-%, bzw. bis 20 Gew.-%, vielfach 0,3 bis 8 Gew.-%, häufig 0,5 bis 6 Gew.-% und meistens 0,5 bis 5 Gew.-% an feinteiligem Bornitrid zusetzen. Diese Gewichtsanteile gelten aber auch ganz allgemein.

Erfindungsgemäß vorteilhaft ist, dass Bornitrid mit Sauerstoff normalerweise erst bei Temperaturen oberhalb von 700°C reagiert. In neutraler oder reduzierender Atmosphäre sowie unter Vakuum ist es sogar bis zu Temperaturen oberhalb von 1000°C stabil. Es ist daher beim erfindungsgemäßen Verfahren vergleichsweise einfach, die thermische Behandlung bei Temperaturen durchzuführen, die unterhalb der vorgenannten Zersetzungs- bzw. Reaktionstemperaturen liegen. In der Regel liegen sie wenigstens 30°C, oder wenigstens 50°C, oder wenigstens 75°C, oder wenigstens 100°C unterhalb der vorgenannten Zersetzungs- bzw. Reaktionstemperaturen.

Erfindungsgemäß wesentlich ist, dass das im erfindungsgemäßen Katalysatorformkörper bei dessen Herstellung verbleibende Bornitrid sich bezüglich der überwiegenden Mehrzahl der bekannten heterogen katalysierten Gasphasenreaktionen weitgehend inert verhält und die katalytischen Eigenschaften der Multielementoxidmasse im wesentlichen nicht beeinträchtigt. Dabei erweist es sich auch als vorteilhaft, dass Bornitrid gegenüber nahezu allen Metallen nicht reaktionsfähig ist. Auch ist vorteilhaft, dass die Oxidationstemperatur von Bornitrid im Unterschied zu Graphit, durch die im Katalysatorvorläuferformkörper vorliegende Umgebung nur unwesentlich beeinflusst wird.

Für das erfindungsgemäße Verfahren ist es ferner von Vorteil, dass Bornitrid im Unterschied zu Graphit seine Gleitmitteleigenschaften auch bei Temperaturen > 400°C nicht verliert. Dies wirkt sich vorteilhaft auf den Zusammenhalt innerhalb der Katalysatorformkörper aus. Weiterhin erweist es sich für das erfindungsgemäße Verfahren als günstig, dass die Massendichte (ca. 2,25 g/cm³ bei 25°C, 1 atm) von Bornitrid vergleichsweise gering und die thermische Leitfähigkeit vergleichsweise hoch ist. Von besonderer Bedeutung für das erfindungsgemäße Verfahren ist außerdem der geringe thermische Ausdehnungskoeffizient von Bornitrid (10⁻⁶/°C im Temperaturbereich 20 bis 1000°C; vgl. Chemical Economy & Engineering Review, Jan. & Feb. 1976 Vol.8, No. 1,2 (No. 92), Seiten 29-34), der sich bei der thermischen Behandlung des Katalysatorvorläuferformkörpers formerhaltend auswirkt. Infolge seiner ausgezeichneten Korrosionsbeständigkeit bleibt das in den Katalysatorformkörpern enthaltene Bornitrid auch im Verlauf der späteren heterogen katalysierten Gasphasenreaktion weitestgehend erhalten. Die Herstellung von Bornitrid wird beispielsweise in der DE-A 24 61 821 beschrieben. Alpha-Bornitrid ist die erfindungsgemäß bevorzugte hexagonale Modifikation (vgl. auch Radex-Rundschau, Schwetz-Reinmuth-Lipp: Refraktäre Borverbindungen, Heft 3, 1981, Seiten 568-585).

Für das erfindungsgemäße Verfahren zweckmäßige Bornitride sind wie folgt beschaffen:

| | |
|---|---|
| Partikeldurchmesser | 1 bis 10 µm, vorzugsweise bis 5 µm; |
| spezifische BET-Oberfläche: | 5 bis 20 m²/g; vorzugsweise bis 15 m²/g. |
| Schüttdichte (bulk density): | 0,2 bis 0,6 g/cm³; |
| Klopfdichte (Tap density): | 0,3 bis 0,7 g/cm³. |

Zusammenfassend weist das erfindungsgemäß zu verwendende Bornitrid eine höhere Oxidationsbeständigkeit als Graphit auf, und ist gleichzeitig in gleicher Weise wie Graphit ausreichend chemisch inert, um die Katalysatorqualität nicht negativ zu beeinträchtigen.

Dem entsprechend können auch die nachfolgenden hexagonalen Bornitridpulver S1, S2 und SX der Firma Elektroschmelzwerk Kempten GmbH, Werke Kempten, zur Durchführung des erfindungsgemäßen Verfahrens eingesetzt werden.

Diese Pulver sind durch die folgenden Eigenschaften gekennzeichnet:

| | S1 | S2 | SX |
|---|---|---|---|
| Reinheit (B+N) | > 98,5 Gew.-% | > 98,5 Gew.-% | > 90,0 Gew.-% |
| Stickstoff (N) | > 54,5 Gew.-% | > 54,5 Gew.-% | > 50,0 Gew.-% |
| Sauerstoff gesamt (O) | < 1,5 Gew.-% | < 1,5 Gew.-% | < 7,0 Gew.-% |
| B₂O₃ | < 0,1 Gew.-% | < 0,1 Gew.-% | < 7,0 Gew.-% |
| Kohlenstoff (C) | < 0,1 Gew.-% | < 0,1 Gew.-% | < 0,1 Gew.-% |
| Metallische Verunreinigungen | < 0,2 Gew.-% | < 0,2 Gew.-% | < 0,2 Gew.-% |
| Kerngröße (Druchmesser) (Primärteilchen) | 3 µm | 5 µm | 3 µm |
| Spezifische Oberfläche (BET, m²/g) | 10 bis 15 | 5 bis 10 | 8 bis 12 |

Besonders vorteilhaft für das erfindungsgemäße Verfahren ist auch die Säurebeständigkeit der in dieser Schrift aufgeführten Bornitride. Eignen sich damit erfindungsgemäß hergestellte Katalysatorformkörper doch insbesondere als Katalysatoren für die Herstellung von α, β-ethylenisch ungesättigten Carbonsäuren durch heterogen katalysierte Partialoxidation geeigneter Vorläuferverbindungen.

Erfindungsgemäß vorteilhaft wird das feinteilige Vorläufergemisch ausschließlich Bornitrid als Formungshilfsmittel enthalten. Selbstverständlich kann das Bornitrid beim erfindungsgemäßen Verfahren aber auch gemeinsam mit anderen Formungshilfsmitteln angewendet werden. Solche anderen Formungshilfsmittel können beispielsweise Ruß, Stearinsäure, Stärke, Polyacrylsäure, Mineral- oder Pflanzenöl, Wasser, feinteiliges Teflonpulver (z.B. Pulver der Fa. Aldrich 43093-5), Bortrifluorid und/oder Graphit sein. Werden Formungshilfsmittelgemische verwendet, gilt für deren Zusatzmenge zum feinteiligen Vorläufergemisch das im Zusammenhang mit den Bornitridzusatzmengen Gesagte.

Die thermische Behandlung der Katalysatorvorläuferformkörper kann erfindungsgemäß vorteilhaft und an die individuelle Art der jeweils angestrebten Aktivmasse angepasst, bei Temperaturen im Bereich von 150°C bis 650°C erfolgen. Häufig wird die thermische Behandlung der Katalysatorvorläuferformkörper bei Temperaturen im Bereich von 200°C bis 600°C bzw. 250°C bis 550°C, bzw. 300°C bis 500°C erfolgen. Die Dauer der thermischen Behandlung kann sich dabei über einen Zeitraum von wenigen Stunden bis zu mehreren Tagen erstrecken. Die thermische Behandlung kann dabei unter Vakuum, unter inerter Atmosphäre (z.B. N₂, Edelgase etc.), unter reduzierender Atmosphäre (z.B. H₂ oder NH₃) oder unter oxidierender Atmosphäre erfolgen. In der Regel werden oxidierende Atmosphären molekularen Sauerstoff enthalten. Typische oxidierende Atmosphären sind Gemische aus Inertgas (N₂, Edelgase etc.) und molekularem Sauerstoff. Üblicherweise wird der Gehalt an molekularem Sauerstoff dabei wenigstens 0,1 Vol.-%, häufig wenigstens 0,2 Vol.-%, vielfach wenigstens 0,5 Vol.-%, oft wenigstens 1 Vol.-%, oder wenigstens 10 Vol.-%, oder wenigstens 20 Vol.-% betragen. Selbstverständlich kann der Gehalt an molekularem Sauerstoff in solchen Gemischen aber auch 30 Vol.-%, oder 40 Vol.-%, oder 50 Vol-%, oder 70 Vol-% oder mehr betragen.

Selbstverständlich kommt als Atmosphäre für die thermische Behandlung aber auch reiner molekularer Sauerstoff in Betracht. Häufig wird die thermische Behandlung unter Luft erfolgen. Generell kann die thermische Behandlung der Katalysatorvorläuferformkörper unter stehender oder unter fließender Gasatmosphäre erfolgen. Der Begriff der Atmosphäre (bzw. der Gasatmosphäre) in welcher die thermische Behandlung erfolgt ist dabei in dieser Schrift so zu verstehen, dass er sich aus den Katalysatorvorläuferformkörpern im Rahmen der thermischen Behandlung aufgrund von Zersetzungsvorgängen entwickelnde Gase nicht umfasst. Selbstverständlich kann die Gasatmosphäre in welcher die thermische Behandlung erfolgt aber auch ausschließlich oder teilweise aus diesen Gasen bestehen. Im Rahmen der bei dem erfindungsgemäßen Verfahren erfolgenden thermischen Behandlung können sowohl die Behandlungstemperatur, als auch die Behandlungsatmosphäre über die Behandlungsdauer zeitlich konstant oder auch zeitlich variabel gestaltet sein.

Die Partikeldurchmesser des feinteiligen Vorläufergemischs (das zugesetzte Formungshilfsmittel ausgenommen) werden bei seiner Formung zur gewünschten Geometrie des Katalysatorvorläuferformkörper in der Regel im Bereich von 10 bis 2000 µm liegen. Vielfach werden vorgenannte Partikeldurchmesser im Bereich 20 bis 1800 µm, oder 30 bis 1700 µm, oder 40 bis 1600 µm, oder 50 bis 1500 µm liegen. Besonders häufig werden diese Partikeldurchmesser 100 bis 1500 µm, oder 150 bis 1500 µm betragen (der Begriff Partikeldurchmesser meint auch hier die längste direkte Verbindung zweier auf der Partikeloberfläche befindlicher Punkte).

Im Regelfall erfolgt die Formung (Verdichtung) des feinteiligen Vorläufergemischs zur Geometrie des Katalysatorvorläuferformkörpers durch Einwirkung äußerer Kräfte (Druck) auf das feinteilige Vorläufergemisch. Der dabei anzuwendende Formgebungsapparat bzw. die dabei anzuwendende Formgebungsmethode unterliegt dabei keiner Beschränkung. Ebenso unterliegt die angestrebte Geometrie des Katalysatorvorläuferformkörpers keiner Beschränkung. D.h., die Katalysatorvorläuferformkörper können regelmäßig oder unregelmäßig geformt sein, wobei regelmäßig geformte Formkörper in der Regel bevorzugt sind.

Häufig wird der Katalysatorvorläuferformkörper Kugelgeometrie aufweisen. Dabei kann der Kugeldurchmesser z.B. 2 bis 10 mm, oder 4 bis 8 mm betragen. Die Geometrie des Katalysatorvorläuferformkörpers kann aber auch vollzylindrisch oder hohlzylindrisch sein. In beiden Fällen können Aussendruchmesser und Länge z.B. 2 bis 10 mm oder 4 bis 8 mm betragen. Im Fall von Hohlzylindern ist in der Regel eine Wandstärke von 1 bis 3 mm zweckmäßig. Selbstverständlich kommen als Katalysatorvorläufergeometrie aber auch alle diejenigen Geometrien in Betracht, die in der WO 02/062737 offenbart und empfohlen werden. In der Regel weicht beim erfindungsgemäßen Verfahren die Geometrie des resultierenden Katalysatorformkörpers von der Geometrie des Katalysatorvorläuferformkörpers nur unwesentlich ab.

An dieser Stelle soll festgehalten werden, dass erfindungsgemäß besonders vorteilhafte Verfahren zur Herstellung von Katalysatorformkörpern und damit besonders günstige Katalysatorformkörper dann resultieren, wenn man in den in den Schriften WO 03/78310, DE-A 198 55 913, WO 02/24620, DE-A 199 22 113, US-A 2005/0131253, WO 02/062737 und WO 05/030393 offenbarten Herstellverfahren an den Stellen, wo feinteiliger Graphit mitverwendet wird, denselben durch gleiche Gewichtsmengen an feinteiligem Bornitrid ersetzt und alle übrigen Herstellmaßnahmen unverändert beibehält. Die dabei resultierenden Katalysatorformkörper sind dann in völlig entsprechender Weise wie in den Schriften DE-A 199 22 113, DE-A 198 55 913, US-A 2005/0131253, WO 02/24620, WO 03/078310, WO 02/062737 und WO 05/030393 beschrieben für die entsprechenden heterogen katalysierten Gasphasenreaktionen einsetzbar. Sie sind dabei insbesondere dann vorteilhaft, wenn das Graphit jeweils durch Boron Nitride Grade A 01 (Number PD-5006, Issue 0-07.99 der Firma H.C. Starck) ersetzt wird.

Die Formgebung kann beim erfindungsgemäßen Verfahren z.B. durch Strangpressen, Tablettieren oder Extrudieren erfolgen. Dabei wird das feinteilige Vorläufergemisch üblicherweise anfasstrocken eingesetzt. Es kann jedoch bis zu 10% seines Gesamtgewichtes Substanzen zugesetzt enthalten, die bei Normalbedingungen (25 °C, 1 atm) flüssig sind. Das erfindungsgemäße Verfahren ist aber auch dann anwendbar, wenn das feinteilige Vorläufergemisch überhaupt keine solchen flüssigen Substanzen mehr enthält. Selbstverständlich kann das feinteilige Vorläufergemisch aber auch aus festen Solvaten (z.B. Hydraten) bestehen, die solche flüssigen Substanzen in chemisch und/oder physikalisch gebundener Form aufweisen.

Die im Rahmen des erfindungsgemäßen Verfahrens angewandten Formgebungsdrucke werden im allgemeinen 50 kg/cm² bis 5000 kg/cm² betragen. Vorzugsweise betragen die Formgebungsdrucke 200 bis 3500 kg/cm², besonders bevorzugt 600 bis 2500 kg/cm². Das vorgenannte gilt insbesondere dann, wenn als Formgebungsverfahren die Tablettierung angewendet wird. Die Grundzüge des Tablettierens sind z.B. in "Die Tablette", Handbuch der Entwicklung, Herstellung und Qualitätssicherung, W.A. Ritschel und A. Bauer-Brandl, 2. Auflage, Editio Cantor Verlag Aulendorf, 2002 beschrieben und in völlig entsprechender Weise auf ein erfindungsgemäßes Tablettierverfahren übertragbar.

Als Multielementoxidmassen kommen im Rahmen des erfindungsgemäßen Verfahrens sowohl Aktivmassen in Betracht, die neben Sauerstoff sowohl Metalle als auch Nichtmetalle als elementare Konstituenten enthalten. Häufig handelt es sich bei den Multielementoxidaktivmassen aber um reine Multimetalloxidaktivmassen.

Für eine Anwendung des erfindungsgemäßen Verfahrens besonders günstige Multielementoxidaktivmassen samt zugehöriger Vorläufermassen sind z.B. jene, die in den Schriften WO 2005/030393, EP-A 467 144, EP-A 1 060 792, DE-A 198 55 913, WO 03/078310, DE-A 199 22 113, WO 02/24620, WO 02/062737 und US-A 2005/0131253 offenbart sind.

Erfindungsgemäß verwendbare feinteilige Vorläufergemische sind in einfachster Weise z.B. dadurch erhältlich, indem man von Quellen der elementaren Konstituenten der angestrebten Aktivmasse ein feinteiliges, möglichst inniges, der Stöchiometrie der gewünschten Aktivmasse entsprechend zusammengesetztes, formbares Gemisch erzeugt, dem noch Formungs- und gegebenenfalls Verstärkungshilfsmittel zugesetzt werden können (oder von Anfang an eingearbeitet werden können).

Als Quellen für die elementaren Konstituenten der gewünschten Aktivmasse kommen grundsätzlich solche Verbindungen in Betracht, bei denen es sich bereits um Oxide handelt und/oder um solche Verbindungen, die durch Erhitzen, wenigstens in Anwesenheit von gasförmigem molekularem Sauerstoff, in Oxide überführbar sind. Prinzipiell kann die Sauerstoffquelle aber auch z.B. in Form eines Peroxids Bestandteil des Vorläufergemisches sein. Auch kann das Vorläufergemisch Verbindungen wie NH₄OH, (NH₄)₂CO₃, NH₄NO₃, NH₄CHO₂, CH₃COOH, NH₄CH₃CO₂ und/oder Ammoniumoxalat zugesetzt enthalten, die bei der thermischen Behandlung als Porenbildner zu vollständig gasförmig entweichenden Verbindungen zerfallen und/oder zersetzt werden können.

Das, vorzugsweise innige, Vermischen der Ausgangsverbindungen (Quellen) zur Herstellung des feinteiligen formbaren Vorläufergemischs, kann beim erfindungsgemäßen Verfahren in trockener oder in nasser Form erfolgen. Erfolgt es in trockener Form, so werden die Ausgangsverbindungen zweckmäßigerweise als feinteilige Pulver (Partikeldurchmesser zweckmäßig im Bereich von 1 bis bzw. 10 bis 2000 µm, vorzugsweise 20 bis 1800 µm, besonders bevorzugt 30 bis 1700 µm und ganz besonders bevorzugt 40 bis 1600 µm, oder 50 bis 1500 µm bzw. 100 bis 1500 µm, oder 150 bis 1500 µm liegend) eingesetzt. Nach Zusatz der erfindungsgemäßen Formungshilfsmittel sowie gegebenenfalls erfolgendem Zusatz weiterer Formungs- und/oder Verstärkerhilfsmittel kann anschließend die Formgebung erfolgen. Solche Verstärkungshilfsmittel können beispielsweise Mikrofasern aus Glas, Asbest, Siliciumcarbid und/oder Kaliumtitanat sein. Ganz generell kann beim erfindungsgemäßen Verfahren eine Ausgangsverbindung Quelle für mehr als einen elementaren Konstituenten sein.

Erfindungsgemäß bevorzugt erfolgt das innige Vermischen jedoch in nasser Form. Üblicherweise werden dabei die Ausgangsverbindungen z.B. in Form einer wässrigen Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene Lösung oder Suspension getrocknet, wobei der Trocknungsprozess vorzugsweise durch Sprühtrocknung mit Austrittstemperaturen von 100 bis 150°C erfolgt. Die Körnung des resultierenden Sprühpulvers beträgt in typischer Weise 20 bis 50 µm. War Wasser die Basis des flüssigen Mediums, wird das resultierende Sprühpulver normalerweise nicht mehr als 20% seines Gewichtes, vorzugsweise nicht mehr als 15% seines Gewichtes und besonders bevorzugt nicht mehr als 10 Gew.-% seines Gewichtes an Wasser enthalten. Diese Prozentsätze gelten in der Regel auch bei Anwendung anderer flüssiger Lösungs- bzw. Suspendierhilfsmittel. Nach Zusatz der erfindungsgemäßen Formungshilfsmittel sowie gegebenenfalls weiterer Formungs- und/oder Verstärkungsmittel, kann das pulverförmige Gemisch als feinteiliges Vorläufergemisch erfindungsgemäß zum gewünschten Katalysatorvorläuferformkörper verdichtet (geformt) werden. Die feinteiligen Formungs- und/oder Verstärkerungshilfsmittel können aber auch bereits vorab der Sprühtrocknung (teilweise oder vollständig) zugesetzt werden.

Eine nur teilweise Entfernung des Lösungs- bzw. Suspendiermittels kann auch dann zweckmäßig sein, wenn seine Mitverwendung als Formungshilfsmittel beabsichtigt ist.

Anstatt das unmittelbar auf dem Sprühpulver fußende feinteilige Vorläufergemisch zu formen, ist es häufig zweckmäßig, zunächst eine Zwischenkompaktierung durchzuführen, um das Pulver zu vergröbern (in der Regel auf Partikeldurchmesser von 100 bis 2000 µm, bevorzugt 150 bis 1500 µm, besonders bevorzugt 400 bis 1000 µm).

Dabei kann bereits vor der Zwischenkompaktierung erfindungsgemäß zu verwendendes Bornitrid als Kompaktierhilfsmittel zugesetzt werden. Anschließend erfolgt auf der Grundlage des vergröberten Pulvers die eigentliche Formgebung, wobei bei Bedarf zuvor nochmals feinteiliges erfindungsgemäßes Bornitrid (sowie gegebenenfalls weitere Formungs- und/oder Verstärkungshilfsmittel) zugegeben werden kann.

Selbstverständlich können als Quellen der elementaren Konstituenten auch Ausgangsverbindungen eingesetzt werden, die ihrerseits durch thermische Behandlung von Vorläuferformkörpern erzeugt wurden, und multielementoxidischer Natur sind. Insbesondere können die Ausgangsverbindungen der elementaren Konstituenten multimetallischer Natur sein.

Das erfindungsgemäße Verfahren eignet sich insbesondere zur Herstellung von Katalysatorformkörpern, deren Aktivmasse ein Multielementoxid ist, innerhalb dessen das Element Mo das numerisch (molar gerechnet) am häufigsten auftretende Element ist. Insbesondere eignet es sich zur Herstellung von Katalysatorformkörpern, deren Aktivmasse ein Multielementoxid ist, das die Elemente Mo, Fe und Bi, oder die Elemente Mo und V, oder die Elemente Mo, V und P umfasst. Die ersteren Katalysatorformkörper in der vorgenannten Reihe eignen sich vor allem für heterogen katalysierte partielle Gasphasenoxidationen von Propylen zu Acrolein. Die zweitgenannten Katalysatorformkörper eignen sich vor allem für heterogen katalysierte partielle Gasphasenoxidationen von Acrolein zur Acrylsäure und die letztgenannten Katalysatorformkörper in der vorgenannten Reihe eignen sich vor allem für heterogen katalysierte partielle Gasphasenoxidationen von Methacrolein zu Methacrylsäure.

Im besonderen umfasst die vorliegende Erfindung ein Verfahren zur Herstellung von ringförmigen Katalysatorformkörpern (auch Vollkatalysatoren genannt, da sie keinen inerten Trägerkörper aufweisen, auf den die Aktivmasse aufgetragen ist) mit gekrümmter und/oder nicht gekrümmter Stirnfläche der Ringe, deren Aktivmasse (in der Aktivmasse enthaltenes Bornitrid ist dabei wie stets in dieser Schrift außer acht gelassen, da es sich im Normalfall chemisch inert verhält und nicht katalytisch aktiv ist) eine Stöchiometrie der allgemeinen Formel I,

Mo₁₂BiₐFe_{b}X¹_{c}X²_{d}X³ₑX⁴_{f}Oₙ (I),

mit
- X¹ =: Nickel und/oder Kobalt,
- X² =: Thallium, ein Alkalimetall und/oder ein Erdalkalimetall,
- X³ =: Zink, Phosphor, Arsen, Bor, Antimon, Zinn, Cer, Blei und oder Wolfram,
- X⁴ =: Silicium, Aluminium, Titan und/oder Zirkonium,

- a =: 0,2 bis 5,
- b =: 0,01 bis 5,
- c =: 0 bis 10,
- d =: 0 bis 2,
- e =: 0 bis 8,
- f =: 0 bis 10 und
- n =: eine Zahl, die durch die Wertigkeit und Häufigkeit der von Sauerstoff verschiedenen Elemente in I bestimmt wird,
oder eine Stöchiometrie der allgemeinen Formel II,

[Y¹_{a'}Y²_{b'}O_{x'}]ₚ[Y³_{c'}Y⁴_{d'}Y⁶_{f'}Y⁷_{g'}Y²_{h'}O_{y'}]_{q} (II),

mit
- Y¹ =: nur Wismut oder Wismut und wenigstens eines der Elemente Tellur, Antimon, Zinn und Kupfer,
- Y² =: Molybdän, oder Wolfram, oder Molybdän und Wolfram,
- Y³ =: ein Alkalimetall, Thallium und/oder Samarium,
- Y⁴ =: ein Erdalkalimetall, Nickel, Kobalt, Kupfer, Mangan, Zink, Zinn, Cadmium und/oder Quecksilber,
- Y⁵ =: Eisen oder Eisen und wenigstens eines der Elemente Vanadium, Chrom und Cer,
- Y⁶ =: Phosphor, Arsen, Bor und/oder Antimon,
- Y⁷ =: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

- a' =: 0,01 bis 8,
- b' =: 0,1 bis 30,
- c' =: 0 bis 4,
- d' =: 0 bis 20
- e' >: 0 bis 20,
- f' =: 0 bis 6,
- g' =: 0 bis 15,
- h' =: 8 bis 16,

- x', y' =: Zahlen, die durch die Wertigkeit und Häufigkeit der von Sauerstoff verschiedenen Elemente in II bestimmt werden und
- p, q =: Zahlen, deren Verhältnis p/q 0,1 bis 10 beträgt,
und deren ringförmige Geometrie, ohne Berücksichtigung einer gegebenenfalls bestehenden Krümmung der Stirnfläche, eine Länge L von 2 bis 11 mm, einen Außendurchmesser A von 2 bis 11 mm und eine Wandstärke W von 0,75 mm bis 1,75 mm aufweist.

Bei diesem Verfahren wird man aus Quellen der elementaren Konstituenten der Aktivmasse ein feinteiliges formbares Vorläufergemisch erzeugen und aus diesem Gemisch, nach Zugabe von erfindungsgemäßem Formungshilfsmittel sowie gegebenenfalls weiterer Formungs- und/oder Verstärkungshilfsmittel, ringförmige Vollkatalysatorvorläuferformkörper formen, deren Stirnflächen gekrümmt und/oder nicht gekrümmt sind, und diese durch thermisches Behandeln bei erhöhter Temperatur in die ringförmigen Vollkatalysatoren überführen.

Außerdem betrifft die vorliegende Erfindung die Verwendung der nach dem erfindungsgemäßen Verfahren erhältlichen ringförmigen Vollkatalysatoren als Katalysatoren mit erhöhter Aktivität und Selektivität für die gasphasenkatalytische Partialoxidation von Propen zu Acrolein sowie von iso-Buten bzw. tert.-Butanol bzw. dessen Methylether zu Methacrolein.

Das vorgenannte Verfahren zur Herstellung der ringförmigen Katalysatorformkörper ist dann besonders vorteilhaft, wenn die Formung (Verdichtung) des feinteiligen Vorläufergemisches so erfolgt, dass die Seitendruckfestigkeit der resultierenden ringförmigen Vollkatalysatorvorläuferformkörper ≥ 10 und ≤ 40 N, besser ≥ 10 und ≤ 35 N, noch besser ≥ 12 N und ≤ 23 N beträgt. Vorzugsweise beträgt die Seitendruckfestigkeit der resultierenden ringförmigen Vollkatalysatorvorläuferformkörper ≥ 13 N und ≤ 22 N, bzw. ≥ 14 N und ≤ 21 N. Ganz besonders bevorzugt beträgt die Seitendruckfestigkeit der resultierenden ringförmig Vollkatalysatorvorläuferformkörper ≥ 15 N und ≤ 20 N.

Ferner beträgt für diese Katalysatortypen die Körnung (der Partikeldurchmesser) des feinteiligen Vorläufergemischs (ausgenommen die zuzusetzenden Hilfsmittel) vorteilhaft 200 µm bis 1500 µm, besonders vorteilhaft 400 µm bis 1000 µm. In günstiger Weise liegen wenigstens 80 Gew.-%, besser wenigstens 90 Gew.-% und besonders vorteilhaft wenigstens 95 oder 98 oder mehr Gew.-% des feinteiligen Vorläufergemischs in diesem Körnungsbereich. Als Seitendruckfestigkeit wird dabei in dieser Schrift die Druckfestigkeit bei Stauchung des ringförmigen Vollkatalysatorvorläuferformkörpers senkrecht zur zylindrischen Einhüllenden (d.h., parallel zur Fläche der Ringöffnung) verstanden. Dabei beziehen sich alle Seitendruckfestigkeiten dieser Schrift auf eine Bestimmung mittels einer Material-Prüfmaschine der Firma Zwick GmbH & Co (D-89079 Ulm) des Typs Z 2.5/TS15. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500 N einsetzbar (relative Messunsicherheit: ± 0,2 %).

Die Messungen wurden mit folgenden Parametern durchgeführt:
Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor die Fläche der zylindrischen Einhüllenden des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Voilkatalysatorvorläuferformkörper Rissbildung zeigt, ist die Seitendruckfestigkeit (SDF).

Erfindungsgemäß besonders vorteilhafte Vollkatalysatorringgeometrien erfüllen zusätzlich die Bedingung L / A = 0,3 bis 0,7. Besonders bevorzugt ist L / A 0,4 bis 0,6.

Weiterhin ist es für die relevanten Vollkatalysatorringe vorteilhaft, wenn das Verhältnis I / A (wobei I der Innendurchmesser der Vollkatalysatorringgeometrie ist) 0,5 bis 0,8, vorzugsweise 0,6 bis 0,7 beträgt.

Besonders vorteilhaft sind Vollkatalysatorringgeometrien, die gleichzeitig eines der vorteilhaften L / A-Verhältnisse und eines der vorteilten I / A-Verhältnisse aufweisen. Solche mögliche Kombinationen sind z.B. L / A = 0,3 bis 0,7 und I / A = 0,5 bis 0,8 oder 0,6 bis 0,7. Alternativ kann L / A 0,4 bis 0,6 und I / A gleichzeitig 0,5 bis 0,8 oder 0,6 bis 0,7 betragen.

Ferner ist es für die relevanten Vollkatalysatorringe bevorzugt, wenn L 2 bis 6 mm und besonders bevorzugt wenn L 2 bis 4 mm beträgt.

Weiterhin ist es vorteilhaft, wenn A 4 bis 8 mm, vorzugsweise 5 bis 7 mm beträgt.

Die Wandstärke der erfindungsgemäß erhältlichen relevanten Vollkatalysatorringgeometrien beträgt mit Vorteil 1 bis 1,5 mm.

D.h., günstige besagte Vollkatalysatorringgeometrien sind z.B. solche mit L = 2 bis 6 mm und A = 4 bis 8 mm oder 5 bis 7 mm. Alternativ kann L 2 bis 4 mm und A gleichzeitig 4 bis 8 mm oder 5 bis 7 mm betragen. In allen vorgenannten Fällen kann die Wandstärke W 0,75 bis 1,75 mm oder 1 bis 1,5 mm betragen.

Besonders bevorzugt sind unter den vorgenannten günstigen Vollkatalysatorgeometrien jene, bei denen gleichzeitig die vorstehend genannten L / A sowie I / A Kombinationen erfüllt sind.

Mögliche relevante Vollkatalysatorringgeometrien sind somit (A x L x I) 5 mm x 3 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm.

Die Stirnflächen der wie beschrieben beschaffenen Ringe können auch entweder beide oder nur eine wie in der EP-A 184790 beschrieben gekrümmt sein und zwar z.B. so, dass der Radius der Krümmung vorzugsweise das 0,4 bis 5-fache des Außendurchmessers A beträgt. Erfindungsgemäß bevorzugt sind beide Stirnflächen ungekrümmt.

Alle diese Vollkatalysatorringgeometrien eignen sich z.B. sowohl für die gasphasenkatalytische Partialoxidation von Propen zu Acrolein als auch für die gasphasenkatalytische Partialoxidation von iso-Buten oder tert.-Butanol oder dem Methylether des tert.-Butanols zu Methacrolein.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a beträgt vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist X¹ vorzugsweise Kobalt, X² ist vorzugsweise K, Cs und/oder Sr, besonders bevorzugt K, X³ ist bevorzugt Zink und/oder Phosphor und X⁴ ist bevorzugt Si. Besonders bevorzugt weisen die Variablen X¹ bis X⁴ gleichzeitig die vorgenannten Bedeutungen auf.

Besonders bevorzugt weisen alle stöchiometrischen Koeffizienten a bis f und alle Variablen X¹ bis X⁴ gleichzeitig ihre vorgenannten vorteilhaften Bedeutungen auf.

Innerhalb der Stöchiometrien der allgemeinen Formel II sind jene bevorzugt, die der allgemeinen Formel III

[Bi_{a"}Z²_{b"}O_{x"}]_{p"}[Z²₁₂Z³_{c"}Z⁴_{d"}Fe_{e"}Z⁵_{f"}Z⁶_{g"}Z⁷_{h"}O_{y"}]_{q"} (III),

mit
- Z² =: Molybdän, oder Wolfram, oder Molybdän und Wolfram,
- Z³ =: Nickel und/oder Kobalt, vorzugsweise Ni oder Co,
- Z⁴ =: Thallium, ein Alkalimetall und/oder ein Erdalkalimetall, vorzugsweise K, Cs und/oder Sr,
- Z⁵ =: Phosphor, Arsen, Bor, Antimon, Zinn, Cer und/oder Bi,
- Z⁶ =: Silicium, Aluminium, Titan und/oder Zirkonium, vorzugsweise Si,
- Z⁷ =: Kupfer, Silber und/oder Gold,

- a" =: 0,1 bis 1,
- b" =: 0,2 bis 2,
- c" =: 3 bis 10,
- d" =: 0,02 bis 2,
- e" =: 0,01 bis 5, vorzugsweise 0,1 bis 3,
- f" =: 0 bis 5,
- g" =: 0 bis 10, vorzugsweise > 0 bis 10, besonders bevorzugt 0,2 bis 10 und ganz besonders bevorzugt 0,4 bis 3,
- h" =: 0 bis 1,
- x", y" =: Zahlen, die durch die Wertigkeit und Häufigkeit der von Sauerstoff verschiedenen Elemente in III bestimmt werden und
- p", q" =: Zahlen, deren Verhältnis p" / q" 0,1 bis 5, vorzugsweise 0,5 bis 2 beträgt,
entsprechen.

Ferner sind Aktivmassen der Stöchiometrie II bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'} enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte Aktivmassen der Stöchiometrie II sind solche, in denen Y¹ nur Wismut ist.

Innerhalb der Aktivmassen der Stöchiometrie III sind diejenigen erfindungsgemäß bevorzugt, in denen Z²_{b"} = (Wolfram)_{b"} und Z²₁₂ = (Molybdän)₁₂ ist.

Ferner sind für die diskutierten ringförmigen Vollkatalysatoren Aktivmassen der Stöchiometrie III bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Bi_{a"}Z²_{b"}O_{x"} enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der wie beschrieben erhältlichen Aktivmassen der Stöchiometrie II (Aktivmassen der Stöchiometrie III) in den Aktivmassen der Stöchiometrie II (Aktivmassen der Stöchiometrie III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}Q_{x'} ([Bi_{a"}Z²_{b"}O_{x"}]) vorliegen, deren Größtdurchmesser im Bereich 1 nm bis 100 µm liegt.

Als Formungshilfsmittel (Gleitmittel) kommen für das erfindungsgemäße Verfahren der Herstellung der relevanten ringförmigen Katalysatorformkörper neben Bornitrid Ruß, Stearinsäure, Stärke, Polyacrylsäure, Mineral- oder Pflanzenöl, Wasser, Bortrifluorid und/oder Graphit in Betracht. Auch Glycerin und Celluloseether können als weitere Gleitmittel eingesetzt werden. Erfindungsgemäß bevorzugt wird ausschließlich Bornitrid als Formungshilfsmittel zugesetzt. Bezogen auf die zum Vollkatalysatorvorläuferformkörper zu formende Masse werden in der Regel ≤ 10 Gew.-%, meist ≤ 5 Gew.-%, vielfach ≤ 3 Gew.-%, oft ≤ 2 Gew.-% an Bornitrid zugesetzt. Üblicherweise beträgt die vorgenannte Zusatzmenge ≥ 0,5 Gew.-%. Bevorzugt zugesetztes Bornitrid ist das Boron Nitride Grade A 01, Number PD-5006, Issue 0-07.99 der Fa. H. C. Starck.

Ferner können feinteilige Verstärkungsmittel wie Mikrofasern aus Glas, Asbest, Siliciumcarbid oder Kaliumtitanat zugesetzt werden. Die Formung zum ringförmigen Vollkatalysatorvorläuferformkörper kann z.B. mittels einer Tablettierungsmaschine, einer Extrusionsverformungsmaschine oder dergleichen durchgeführt werden.

Die thermische Behandlung des relevanten ringförmigen Vollkatalysatorvorläuferformkörpers erfolgt in der Regel bei Temperaturen, die 350°C überschreiten. Normalerweise wird im Rahmen der thermischen Behandlung die Temperatur von 650°C jedoch nicht überschritten. Erfindungsgemäß vorteilhaft wird im Rahmen der thermischen Behandlung die Temperatur von 600°C, bevorzugt die Temperatur von 550°C und besonders bevorzugt die Temperatur von 500°C nicht überschritten. Ferner wird im Rahmen der thermischen Behandlung des ringförmigen Vollkatalystorvorläuferformkörpers vorzugsweise die Temperatur von 380°C, mit Vorteil die Temperatur von 400°C, mit besonderem Vorteil die Temperatur von 420°C und ganz besonders bevorzugt die Temperatur von 440°C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350°C, vorzugsweise 220 bis 280°C, und daran anschließend eine thermische Behandlung bei einer Temperatur von 400 bis 600°C, vorzugsweise 430 bis 550°C durchgeführt werden.

Normalerweise nimmt die thermische Behandlung des ringförmigen Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringförmigen Vollkatalysatorvorläuferformkörpers Behandlungsdauern von 45 h bzw. 25 h nicht überschritten. Oft liegt die Gesamtbehandlungsdauer unterhalb von 20 h. Erfindungsgemäß vorteilhaft werden im Rahmen der thermischen Behandlung des relevanten ringförmigen Vollkatalysatorvorläuferformkörpers 500°C (460°C) nicht überschritten und die Behandlungsdauer im Temperaturfenster von ≥ 400°C (≥ 440°C) erstreckt sich auf 5 bis 20 h.

Die thermische Behandlung (auch die nachfolgend angesprochene Zersetzungsphase) der ringförmigen Vollkatalysatorvorläuferformkörper kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂ oder Methan, Acrolein, Methacrolein) erfolgen. Selbstredend kann die thermische Behandlung auch unter Vakuum ausgeführt werden.

Prinzipiell kann die thermische Behandlung der relevanten ringförmigen Vollkatalysatorvorläuferformkörper in den unterschiedlichsten Ofentypen wie z.B. beheizbare Umluftkammern, Hordenöfen, Drehrohröfen, Bandcalzinierer oder Schachtöfen durchgeführt werden. Bevorzugt erfolgt die thermische Behandlung der ringförmigen Vollkatalysatorvorläuferformkörper in einer Bandcalciniervorrichtung, wie sie die DE-A 10046957 und die WO 02/24620 empfehlen.

Die thermische Behandlung der relevanten ringförmigen Volikatalysatorvorläuferformkörper unterhalb von 350°C verfolgt in der Regel die thermische Zersetzung der in den Vollkatalysatorvorläuferformkörpern enthaltenen Quellen der elementaren Konstituenten des angestrebten ringförmigen Vollkatalysators. Häufig erfolgt diese Zersetzungsphase im Rahmen des Aufheizens auf Temperaturen ≥ 350°C.

Die ringförmigen Vollkatalysatorvorläuferformkörper von angestrebten ringförmigen Vollkatalysatoren, deren Aktivmasse eine Stöchiometrie der allgemeinen Formel I, oder der allgemeinen Formel II, oder der allgemeinen Formel III aufweist, können dadurch hergestellt werden, dass man von Quellen der elementaren Konstituenten der Aktivmasse des gewünschten ringförmigen Vollkatalysators ein (möglichst inniges) feinteiliges, der Stöchiometrie der gewünschten Aktivmasse entsprechend zusammengesetztes, formbares Gemisch erzeugt und aus diesem, nach Zusatz von (u.a. erfindungsgemäßen) Formungs- und gegebenenfalls Verstärkungshilfsmitteln, einen ringförmigen Vollkatalysatorvorläuferformkörper (mit gekrümmter und/oder nicht gekrümmter Stirnfläche) formt, dessen Seitendruckfestigkeit ≥ 12 N und ≤ 23 N beträgt. Die Geometrie des ringförmigen Vollkatalysatorvorläuferformkörpers wird dabei im wesentlichen derjenigen des gewünschten ringförmigen Vollkatalysators entsprechen.

Als Quellen für die elementaren Konstituenten der gewünschten Aktivmasse kommen solche Verbindungen in Betracht, bei denen es sich bereits um Oxide handelt und/oder um solche Verbindungen, die durch Erhitzen, wenigstens in Anwesenheit von molekularem Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die beim thermischen Behandeln zu vollständig gasförmig entweichenden Verbindungen zerfallen und/oder zersetzt werden können, können in das feinteilige formbare Gemisch (vorzugsweise ein Trockengemisch) zusätzlich eingearbeitet werden).

Das, vorzugsweise innige, Vermischen der Ausgangsverbindungen (Quellen) zur Herstellung des feinteiligen formbaren Gemischs kann beim erfindungsgemäßen Verfahren in trockener oder in nasser Form erfolgen. Erfolgt es in trockener Form, so werden die Ausgangsverbindungen zweckmäßigerweise als feinteilige Pulver (die Körnung sollte vorteilhaft ≤ 100 µm, vorzugsweise ≤ 50 µm betragen; in der Regeln wird der zahlenmittlere Korngrößtdurchmesser ≥ 1 µm bzw. ≥ 10 µm betragen) eingesetzt. Nach Zusatz von (u.a. erfindungsgemäßen) Formungs- und gegebenenfalls Verstärkungshilfsmitteln kann anschließend die Formgebung zum ringförmigen Vollkatalysatorvorläuferformkörper erfolgen.

Bevorzugt erfolgt das innige Vermischen jedoch in nasser Form. Üblicherweise werden dabei die Ausgangsverbindungen in Form einer wässrigen Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene Lösung oder Suspension getrocknet, wobei der Trocknungsprozess vorzugsweise durch Sprühtrocknung mit Austrittstemperaturen von 100 bis 150°C erfolgt. Die Körnung des resultierenden Sprühpulvers beträgt in typischer Weise 20 bis 50 µm.

Das Sprühpulver kann nun nach Zusatz von (u.a. erfindungsgemäßen) Formungs- und gegebenenfalls Verstärkungshilfsmitteln zu den ringförmigen Vollkatalysatorvorläuferformkörpern verdichtet (geformt) werden. Die feinteiligen Formungs- und gegebenenfalls Verstärkungshilfsmittel können aber auch bereits vorab der Sprühtrocknung (teilweise oder vollständig) zugesetzt werden. Auch kann bei der Trocknung das Lösungs- bzw. Suspendiermittel nur teilweise entfernt werden, falls seine Mitverwendung als Formungshilfsmittel beabsichtigt ist.

Anstatt das Sprühpulver nach Zusatz von (u.a. erfindungsgemäßen) Formungs- und gegebenenfalls Verstärkungshilfsmitteln, unmittelbar zu den ringförmigen Vollkatalysatorvorläuferformkörpern (mit gekrümmter und/oder nicht gekrümmter Stirnfläche der Ringe) zu formen, ist es häufig zweckmäßig, zunächst eine Zwischenkompaktierung durchzuführen, um das Pulver zu vergröbern (in der Regel auf eine Körnung von 400 µm bis 1 mm). Anschließend erfolgt mit dem vergröberten Pulver die eigentliche Ringformung, wobei bei Bedarf zuvor nochmals feinteiliges erfindungsgemäßes Gleitmittel zugegeben werden kann.

Eine solche Zwischenkompaktierung zum Zweck der Kornvergröberung kann beispielsweise mittels eines Kompaktierers der Fa. Hosokawa Bepex GmbH (D-74211 Leingarten), vom Typ Kompaktor K 200/100 erfolgen. Die Härte des Zwischenkompaktats liegt häufig bereits im Bereich von 10 N. Für die Ringformung zum Vollkatalysatorvorläuferformkörper kommt z.B. ein Kilian Rundläufer (der Fa. Kilian in D-50735 Köln) vom Typ RX 73 oder S 100 in Betracht. Alternativ kann eine Tablettenpresse der Fa. Korsch (D-13509 Berlin) vom Typ PH 800-65 eingesetzt werden.

Insbesondere zur Herstellung von Aktivmassen der Stöchiometrie der allgemeinen Formel II oder III ist es vorteilhaft, ein Mischoxid Y¹_{a'}Y²_{b'}O_{x'} bzw. Bi_{a"}Z²_{b"}O_{x"} als Quelle der Elemente Y¹, Y² bzw. Bi, Z² in Abwesenheit der übrigen Konstituenten der Aktivmassen der Stöchiometrie der allgemeinen Formel II bzw. III vorzubilden und damit nach seiner Vorbildung, wie bereits beschrieben, mit Quellen der übrigen Konstituenten der Aktivmassen der Stöchiometrie der allgemeinen Formel II bzw. III ein feinteiliges formbares Gemisch zu erzeugen, um daraus, nach Zusatz von (u.a. erfindungsgemäßen) Formungs- und gegebenenfalls Verstärkungshiffsmitteln den ringförmigen Vollkatalysatorvorläuferformkörper zu formen.

Bei einer solchen Vorgehensweise ist lediglich darauf zu achten, dass für den Fall, dass die Herstellung des feinteiligen formbaren Gemischs nass erfolgt (in Suspension), die vorgebildeten Mischoxide Y¹_{a'}Y²_{b'}O_{x'} bzw. Bi_{a"}Z²_{b"}Q_{x"} nicht in nennenswertem Umfang in Lösung gehen.

Ausführlich wird eine wie vorstehend beschriebene Herstellweise in den Schriften DE-A 4407020, EP-A 835, EP-A 575897 und DE-C 3338380 beschrieben.

Beispielsweise kann man wasserlösliche Salze von Y¹ wie Nitrate, Carbonate, Hydroxide oder Acetate mit Y²-Säuren oder deren Ammoniumsalzen in Wasser mischen, die Mischung trocknen (vorzugsweise sprühtrocknen) und die getrocknete Masse anschließend thermisch behandeln. Die thermisch behandelte Masse wird nachfolgend zweckmäßig zerkleinert (z.B. in einer Kugelmühle oder durch Strahlmahlen) und aus dem dabei erhältlichen, in der Regel aus im wesentlichen kugelförmigen Partikeln bestehenden, Pulver die Kornklasse mit einem im für die Aktivmasse der Stöchiometrie der allgemeinen Formel II bzw. III gewünschten Größtdurchmesserbereich liegenden Korngrößtdurchmesser durch in an sich bekannter Weise durchzuführendes Klassieren (z.B. Nass- oder Trockensiebung) abgetrennt und vorzugsweise mit, bezogen auf die Masse dieser abgetrennten Kornklasse, 0,1 bis 3 Gew.-%, feinteiligem SiO₂ (der zahlenmittlere Korngrößtdurchmesser der üblicherweise im wesentlichen kugelförmigen SiO₂-Partikel beträgt zweckmäßigerweise 10 bis 50 nm) vermischt und so eine Ausgangsmasse 1 hergestellt. Die thermische Behandlung erfolgt zweckmäßig bei Temperaturen von 400 bis 900°C, vorzugsweise bei 600 bis 900°C. Letzteres gilt insbesondere dann, wenn es sich bei dem vorgebildeten Mischoxid um ein solches der Stöchiometrie BiZ²O₆, Bi₂Z²₂O₉ und/oder Bi₂Z²₃O₁₂ handelt, unter denen das Bi₂Z²₂O₉ bevorzugt ist, insbesondere wenn Z² = Wolfram.

Üblicherweise erfolgt die thermische Behandlung im Luftstrom (z.B. in einem Drehrohrofen, wie er in der DE-A 10325487 beschrieben ist). Die Dauer der thermischen Behandlung erstreckt sich in der Regel auf wenige Stunden.

Von den übrigen Bestandteilen der gewünschten Aktivmasse der allgemeinen Formel II bzw. III wird normalerweise ausgehend von in an sich bekannter Weise geeigneten Quellen (vgl. EP-A 835 und DE-C 3338380 sowie DE-A 4407020) in erfindungsgemäß zweckmäßiger Weise z.B. ein möglichst inniges, vorzugsweise feinteiliges Trockengemisch hergestellt (z.B. wasserlösliche Salze wie Halogenide, Nitrate, Acetate, Carbonate oder Hydroxide in einer wässrigen Lösung vereinen und anschließend die wässrige Lösung z.B. sprühtrocknen oder nicht wasserlösliche Salze, z.B. Oxide, in wässrigem Medium suspendieren und anschließend die Suspension z.B. sprühtrocknen), das hier als Ausgangsmasse 2 bezeichnet wird. Wesentlich ist nur, dass es sich bei den Bestandteilen der Ausgangsmasse 2 entweder bereits um Oxide handelt, oder um solche Verbindungen, die durch Erhitzen, gegebenenfalls in Anwesenheit von Sauerstoff, in Oxide überführbar sind. Anschließend werden die Ausgangsmasse 1 und die Ausgangsmasse 2 im gewünschten Mengenverhältnis in erfindungsgemäßer Weise, d.h. nach Zusatz von (u.a. erfindungsgemäßen) Formungs- und gegebenenfalls Verstärkungshilfsmitteln, zum ringförmigen Vollkatalysatorvorläuferformkörper formbaren Gemisch vermischt. Die Formung kann, wie bereits beschrieben, anwendungstechnisch zweckmäßig über die Stufe einer Zwischenkompaktierung erfolgen.

In einer weniger bevorzugten Ausführungsform kann das vorgebildete Mischoxid Y¹_{a'}Y²_{b}·Oₓ· bzw. Bi_{a"}Z²_{b"}O_{x"} mit Quellen der übrigen Bestandteile der gewünschten Aktivmasse auch in flüssigem, vorzugsweise wässrigem, Medium innig vermischt werden. Dieses Gemisch wird anschließend z.B. zu einem innigen Trockengemisch getrocknet und sodann, wie bereits beschrieben, geformt und thermisch behandelt. Dabei können die Quellen der übrigen Konstituenten in diesem flüssigen Medium gelöst und/oder suspendiert vorliegen, wohingegen das vorgebildete Mischoxid in diesem flüssigen Medium im wesentlichen unlöslich sein sollte, d.h., suspendiert vorliegen muss.

Die vorgebildeten Mischoxidpartikel sind im fertiggestellten ringförmigen Vollkatalysator in der durch die Klassierung eingestellten Längstausdehnung im wesentlichen unverändert enthalten.

Bevorzugt beträgt die spezifische Oberfläche von solchermaßen vorgebildeten Mischoxiden Y¹_{a'}Y²_{b'}O_{x'} bzw. Bi_{a"}Z²_{b"}O_{x"} 0,2 bis 2, besonders bevorzugt 0,5 bis 1,2 m²/g. Ferner resultiert das Porengesamtvolumen von solchermaßen vorgebildeten Mischoxiden vorteilhaft überwiegend von Mikroporen.

Alle Angaben in dieser Schrift zu Bestimmungen von spezifischen Oberflächen bzw. von Mikroporenvolumina beziehen sich, soweit nichts anderes gesagt wird, auf Bestimmungen nach DIN 66131 (Bestimmung der spezifischen Oberfläche von Feststoffen durch Gasadsorption (N₂) nach Brunauer-Emmet-Teller (BET)).

Alle Angaben in dieser Schrift zu Bestimmungen von Porengesamtvolumina sowie von Durchmesserverteilungen auf diese Porengesamtvolumina beziehen sich, soweit nichts anderes erwähnt wird, auf Bestimmungen mit der Methode der Quecksilberporosimetrie in Anwendung des Gerätes Auto Pore 9220 der Fa. Micromeritics GmbH, 4040 Neuß, DE (Bandbreite 30 Å bis 0,3 mm).

Vorteilhafte relevante ringförmige Vollkatalysatoren sind solche, deren spezifische Oberfläche O 5 bis 20 bzw. 15 m²/g, häufig 5 bis 10 m²/g beträgt. Das Porengesamtvolumen solcher ringförmigen Vollkatalysatoren liegt dabei vorteilhaft im Bereich von 0,1 bis 1 bzw. 0,8 cm³/g, häufig im Bereich 0,2 bis 0,4 cm³/g.

Im Unterschied zur Lehre der WO 03/039744 sowie zur Lehre der EP-A 279374 tragen die verschiedenen Porendurchmesser bei wie beschrieben erhaltenen ringförmigen Vollkatalysatoren in vorteilhafter Weise wie folgt zum Porengesamtvolumen bei:
Poren mit Durchmesser im Bereich < 0,03 µm: ≤ 5 Vol.-%;
Poren mit Durchmesser im Bereich von ≥ 0,03 bis ≤ 0,1 µm: ≤ 25 Vol.-%;
Poren mit Durchmesser im Bereich von > 0,1 bis < 1 µm: ≥ 70 Vol.-%; und
Poren mit Durchmesser im Bereich ≥ 1 bis ≤ 10 µm: ≤ 10 Vol.-%.

D.h., im Unterschied zur Lehre der EP-A 279374, spielt bei wie beschrieben erhaltenen ringförmigen Vollkatalysatoren der Anteil der Poren mit einem Durchmesser ≥ 1 µm in der Regel nur eine untergeordnete Rolle.

Ferner spielt bei wie beschrieben erhaltenen ringförmigen Vollkatalysatoren der Anteil der Poren mit einem Durchmesser im Bereich von ≥ 0,03 bis ≤ 0,1 µm in der Regel eine kleinere Rolle.

Besonders vorteilhaft verteilt sich bei wie beschrieben erhaltenen ringförmigen Vollkatalysatoren der Anteil der verschiedenen Porendurchmesser am Porengesamtvolumen wie folgt:

Poren mit Durchmesser im Bereich < 0,03 µm: ≥ 0 und ≤ 5 Vol.-%, vorzugsweise ≤ 3 Vol.-%;

Poren mit Durchmesser im Bereich von ≥ 0,03 bis ≤ 0,1 µm: ≥ 3 bzw. ≥ 5 und ≤ 20 bzw. ≤ 15 Vol.-%;

Poren mit Durchmesser im Bereich von > 0,1 bis < 1 µm: ≥ 75 bzw. ≥ 80 und ≤ 95 bzw. ≤ 90 Vol.-%;

Poren mit Durchmesser im Bereich ≥ 1 µm bis ≤ 10 µm: ≥ 0 und ≤ 5 Vol.-%, vorzugsweise ≤ 3 Vol.-%.

D.h., für wie beschrieben erhaltene ringförmige Vollkatalysatoren spielt hinsichtlich deren Performance bei der Verwendung als Katalysatoren zur Partialoxidation von Propen zu Acrolein, bzw. iso-Buten oder tert. Butanol oder Methylether von tert. Butanol zu Methacrolein der Porendurchmesserbereich > 0,1 bis < 1 µm die entscheidende Rolle.

Im Unterschied dazu fördern Poren im Porendurchmesserbereich von 0,01 bis 0,1 µm die Partialoxidation von Propen zu Acrylsäure. Dies ist dann vorteilhaft, wenn die Aktivmasse in der ersten Stufe einer zweistufigen Partialoxidation von Propen zu Acrylsäure eingesetzt wird, da in der ersten Stufe gebildete Acrylsäure in der zweiten Stufe weitgehend erhalten bleibt.

Das vorstehende wird auch dadurch zusätzlich bestätigt, dass für wie beschrieben erhaltene, besonders vorteilhafte ringförmige Vollkatalysatoren nicht nur die vorstehenden Bedingungen hinsichtlich spezifischer Oberfläche O, Porengesamtvolumen V und Porendurchmesserverteilung erfüllt sind, sondern zusätzlich der prozentual den größten Beitrag zum Porengesamtvolumen V leistende Porendurchmesser d^{max} im Durchmesserbereich 0,3 bis 0,8 µm, besonders vorteilhaft im Durchmesserbereich 0,4 bis 0,7 µm und ganz besonders vorteilhaft im Durchmesserbereich 0,5 bis 0,6 µm liegt.

Es überrascht, dass mit zunehmender Seitendruckfestigkeit des ringförmigen Vollkatalysatorvorläuferformkörpers die Porendurchmesser im resultierenden Vollkatalysatorring in der Regel zu größeren Werten verschoben werden.

Dies überrascht insofern, als die Seitendruckfestigkeit des resultierenden ringförmigen Vollkatalysators sich dabei gleichzeitig zu höheren Werten verschiebt. In überraschender Weise ist die Seitendruckfestigkeit des wie beschrieben resultierenden ringförmigen Vollkatalysators im Regelfall kleiner als die Seitendruckfestigkeit des zugehörigen ringförmigen Vollkatalysatorvorläuferformkörpers.

In typischer Weise betragen die Seitendruckfestigkeiten von wie beschrieben erhältlichen ringförmigen Vollkatalysatoren 5 bis 13 N, häufig 8 bis 11 N. Diese Seitendruckfestigkeiten von wie beschrieben erhältlichen ringförmigen Vollkatalysatoren liegen normalerweise auch dann vor, wenn die übrigen als vorteilhaft beschriebenen physikalischen Eigenschaften (z.B. O, V und Porendurchmesserverteilung) von wie beschrieben erhältlichen ringförmigen Vollkatalysatoren vorliegen.

Wie bereits erwähnt, eignen sich die wie beschrieben erhältlichen ringförmigen Vollkatalysatoren insbesondere als Katalysatoren für die Partialoxidation von Propen zu Acrolein bzw. von iso-Buten und/oder tert. Butanol zu Methacrolein. Die Partialoxidation kann dabei z.B. wie in den Schriften WO 00/53557, WO 00/53558, DE-A 199 10 506, EP-A 1 106 598, WO 01/36364, DE-A 199 27 624, DE-A 199 48 248, DE-A 199 48 523, DE-A 199 48 241, EP-A 700 714, DE-A 10313213, DE-A 103 13 209, DE-A 102 32 748, DE-A 103 13 208, WO 03/039744. EP-A 279 374, DE-A 33 38 380, DE-A 33 00 044, EP-A 575 897, DE-A 10 2004 003 212, DE-A 10 2005 013 039, DE-A 10 2005 009 891, DE-A 10 2005 010 111, DE-A 10 2005 009 885 sowie DE-A 44 07 020 beschrieben durchgeführt werden, wobei die Katalysatorbeschickung z.B. nur wie beschrieben erhältliche ringförmige Vollkatalysatoren oder z.B. mit inerten Formkörpern verdünnte ringförmige Vollkatalysatoren umfassen kann. Im letzteren Fall wird die Katalysatorbeschickung vorteilhaft in der Regel so gestaltet, dass ihre volumenspezifische Aktivität in Strömungsrichtung der Reaktionsgasgemisches kontinuierlich, sprunghaft und/oder stufenförmig zunimmt.

Dabei erweisen sich insbesondere die in dieser Schrift individualisiert hervorgehobenen Ringgeometrien der wie beschrieben erhältlichen Vollkatalysatoren dann als vorteilhaft, wenn die Belastung der Katalysatorbeschickung mit im Reaktionsgasausgangsgemisch enthaltenem Propen, iso-Buten und/oder tert. Butanol (bzw. dessen Methylether) ≥ 130 Nl/l Katalysatorbeschickung ·h beträgt (Vor- und/oder Nachschüttungen aus reinem Inertmaterial werden bei Belastungsbetrachtungen nicht als zur Katalysatorbeschickung gehörig betrachtet). Dies insbesondere dann, wenn auch die anderen in dieser Schrift als vorteilhaft beschriebenen physikalischen Eigenschaften von wie beschrieben erhältlichen ringförmigen Vollkatalysatoren gegeben sind.

Dieses vorteilhafte Verhalten von wie beschrieben erhältlichen ringförmigen Vollkatalysatoren, insbesondere den vorgenannten, liegt aber auch dann vor, wenn die vorgenannte Belastung der Katalysatorbeschickung ≥ 140 Nl/l·h, oder ≥ 150 Nl/l·h, oder ≥ 160 Nl/l·h beträgt. Im Normalfall wird die vorgenannte Belastung der Katalysatorbeschickung ≤ 600 Nl/l·h, häufig ≤ 500 Nl/l·h, vielfach ≤ 400 Nl/l·h oder ≤ 350 Nl/l·h betragen. Belastungen im Bereich von 160 Nl/l·h bis 300 bzw. 250 oder 200 Nl/l·h sind besonders typisch.

Selbstverständlich können die wie beschrieben erhältlichen ringförmigen Vollkatalysatoren als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von < 130 Nl/l·h, oder ≤ 120 Nl/l·h, oder ≤ 110 Nl/l·h, betrieben werden. In der Regel wird diese Belastung jedoch bei Werten ≥ 60 Nl/l·h, oder ≥ 70 Nl/l·h, oder ≥ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:
a) die Belastung der Katalysatorbeschickung mit Reaktionsgasausgangsgemisch; und/oder
b) den Gehalt des Reaktionsgasausgangsgemischs mit der partiell zu oxidierenden Ausgangsverbindung.

Die erfindungsgemäß erhältlichen ringförmigen Vollkatalysatoren eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. dessen Methyletheranteil)) im Reaktionsgasausgangsgemisch wird im Regelfall (d.h. im wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen).

Häufig wird man das Verfahren der mit den wie beschrieben erhältlichen ringförmigen Vollkatalysatoren katalysierten Partialoxidation (im wesentlichen unabhängig von der Belastung) bei einem partiell zu oxidierende (organische) Verbindung (z.B. Propen): Sauerstoff : indifferente Gase (einschließlich Wasserdampf) Volumenverhältnis im Reaktionsgasausgangsgemisch von 1:(1,0 bis 3,0):(5 bis 25), vorzugsweise 1:(1,5 bis 2,3):(10 bis 15) durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu ≥ 20 Vol.-%, oder zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Bei Belastungen der Katalysatorbeschickung mit der partiell zu oxidierenden organischen Verbindung von ≥ 250 Nl/l·h ist jedoch die Mitverwendung von inerten Verdünnungsgasen wie Propan, Ethan, Methan, Pentan, Butan, CO₂, CO, Wasserdampf und/oder Edelgasen für das Rektionsgasausgangsgemisch empfehlenswert. Generell können diese inerten Gase und ihre Gemische aber auch bereits bei geringeren erfindungsgemäßen Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im wesentlichen selektiv abtrennt. Dabei ist zu berücksichtigen, dass die Partialoxidationen zu Acrolein oder Methacrolein mit den wie beschrieben erhältlichen ringförmigen Vollkatalysatoren nur die erste Stufe einer zweistufigen Partialoxidation zu Acrylsäure oder Meth-acrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Bei der Partialoxidation von Propen zu Acrolein, unter Anwendung der wie beschrieben erhältlichen ringförmigen Vollkatalysatoren, kann eine typische Zusammensetzung des Reaktionsgasausgangsgemischs (unabhängig von der gewählten Belastung) z.B. die nachfolgenden Komponenten enthalten:
6 bis 6,5 Vol.-% Propen,
3 bis 3,5 Vol.-% H₂O,
0,3 bis 0,5 Vol.-% CO,
0,8 bis 1,2 Vol.-% CO₂,
0,025 bis 0,04 Vol.-% Acrolein,
10,4 bis 10,7 Vol.-% O₂ und
als Restmenge ad 100% molekularer Stickstoff,
oder: 5,4 Vol.-% Propen,
10,5 Vol.-% Sauerstoff,
1,2 Vol.-% COₓ,
81,3 Vol.-% N₂ und
1,6 Vol.-% H₂O.

Das Reaktionsgasausgangsgemisch kann aber auch wie folgt zusammengesetzt sein:
6 bis 15 Vol.-% Propen,
4 bis 30 Vol.-% (häufig 6 bis 15 Vol.-%) Wasser,
≥ 0 bis 10 Vol.-% (vorzugweise ≥ 0 bis 5 Vol.-%) von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen
1,5 bis 2,5 beträgt und als Restmenge bis zur 100 Vol.-% Gesamtmenge aus molekularem Stickstoff.

Eine andere mögliche Reaktionsgasausgangsgemischzusammensetzung kann enthalten:
6,0 Vol.-% Propen,
60 Vol.-% Luft und
34 Vol.-% H₂O.

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Auch eignen sich die wie beschrieben erhältlichen ringförmigen Katalysatoren für die Verfahren der DE-A 10246119 bzw. DE-A 10245585.

Weitere erfindungsgemäß geeignete Reaktionsgasausgangsgemische können im nachfolgenden Zusammensetzungsraster liegen:

| | |
|---|---|
| 7 bis 11 Vol.-% | Propen, |
| 6 bis 12 Vol.-% | Wasser, |
| ≥ 0 bis 5 Vol.-% | von Propen, Wasser, Sauerstoff und Stickstoff verschiedenen Bestandteilen, |

soviel molekularem Sauerstoff, dass das molare Verhältnis von enthaltenem Sauerstoff zu enthaltenem molekularem Propen 1,6 bis 2,2 beträgt, und
als Restmenge bis zur 100 Vol.-% Gesamtmenge molekularer Stickstoff.

Im Fall von Methacrolein als Zielverbindung kann das Reaktionsgasausgangsgemisch insbesondere auch wie in der DE-A 44 07 020 beschrieben zusammengesetzt sein.

Die Reaktionstemperatur für die Propenpartialoxidation liegt bei Verwendung der wie beschrieben erhältlichen ringförmigen Vollkatalysatoren häufig bei 300 bis 380°C. Das gleiche trifft im Fall von Methacrolein als Zielverbindung zu.

Der Reaktionsdruck liegt für die vorgenannten Partialoxidationen in der Regel bei 0,5 bzw. 1,5 bis 3 bzw. 4 bar.

Die Gesamtbelastung der Katalysatorbeschickung mit Reaktionsgasausgangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgasausgangsgemisch zu verwendendes Propen kommen vor allem polymer grade Propen und chemical grade Propen in Betracht, wie es z.B. die DE-A 10232748 beschreibt.

Als Sauerstoffquelle wird normalerweise Luft eingesetzt.

Die Partialoxidation in Anwendung der wie beschrieben erhältlichen ringförmigen Vollkatalysatoren kann im einfachsten Fall z.B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Eine typische Kontaktrohrlänge beläuft sich z.B. auf 3,20 m. Anwendungstechnisch zweckmäßig beläuft sich die im Rohrbündelbehälter untergebrachte Anzahl an Kontaktrohren auf wenigstens 1000, vorzugsweise auf wenigstens 5000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl.EP-B 468 290).

Die Partialoxidation kann aber auch in einem Mehrzonen (z.B. "Zwei-Zonen") - Vielkontaktrohr-Festbettreaktor durchgeführt werden, wie es die DE-A 199 10 506, die DE-A 10313213, die DE-A 10313208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z.B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittstelle aus der Temperaturzone um 0 bis 15°C, häufig 1 bis 10°C, oder 2 bis 8°C, oder 3 bis 6°C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598, DE-A 19948523, DE-A 19948248, DE-A 10313209, EP-A 700 714, DE-A 10313208, DE-A 10313213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohr und Arbeitsrohren gleich ist.

Der Druckverlust sollte bei Arbeitsrohren und Thermorohr, bezogen auf gleiche GHSV, gleich sein. Ein Druckverlustangleich beim Thermorohr kann durch Zusatz von versplittetem Katalysator zu den Katalysatorformkörpern erfolgen. Dieser Ausgleich erfolgt zweckmäßig über die gesamte Thermorohrlänge homogen.

Zur Bereitung der Katalysatorbeschickung in den Kontaktrohren können, wie bereits erwähnt, nur wie beschrieben erhältliche ringförmige Vollkatalysatoren oder z.B. auch weitgehend homogene Gemische aus wie beschrieben erhältlichen ringförmigen Vollkatalysatoren und keine Aktivmasse aufweisenden, sich bezüglich der heterogen katalysierten partiellen Gasphasenoxidation im wesentlichen inert verhaltenden Formkörpern verwendet werden. Als Materialien für solche inerten Formkörper kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie die Katalysatorformkörper, Ringe sein. Häufig wird man als inerte Verdünnungsformkörper solche wählen, deren Geometrie derjenigen der mit ihnen zu verdünnenden Katalysatorformkörper entspricht. Längs der Katalysatorbeschickung kann aber auch die Geometrie des Katalysatorformkörpers gewechselt oder Katalysatorformkörper unterschiedlicher Geometrie in weitgehend homogener Abmischung eingesetzt werden. In einer weniger bevorzugten Vorgehensweise kann auch die Aktivmasse des Katalysatorformkörpers längs der Katalysatorbeschickung verändert werden.

Ganz generell wird, wie bereits erwähnt, die Katalysatorbeschickung mit Vorteil so gestaltet, dass die volumenspezifische (d.h., die auf die Einheit des Volumens normierte) Aktivität in Strömungsrichtung des Reaktionsgasgemisches entweder konstant bleibt oder zunimmt (kontinuierlich, sprunghaft oder stufenförmig).

Eine Verringerung der volumenspezifischen Aktivität kann in einfacher Weise z.B. dadurch erzielt werden, dass man eine Grundmenge von erfindungsgemäß einheitlich hergestellten ringförmigen Vollkatalysatoren mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen ringförmigen Vollkatalysatoren so verändert, dass die in der Einheit des Ringgesamtvolumens (einschließlich der Ringöffnung) enthaltene Aktivmassenmenge kleiner wird.

Für die heterogen katalysierten Gasphasenpartialoxidationen mit den wie beschrieben erhältlichen ringförmigen Vollkatalysatoren wird die Katalysatorbeschickung vorzugsweise entweder auf der gesamten Länge einheitlich mit nur einem Vollkatalysatorring gestaltet oder wie folgt strukturiert. Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Katalysatorbeschickung, ein im wesentlichen homogenes Gemisch aus erfindungsgemäß erhältlichem ringförmigem Vollkatalysator und inertem Verdünnungsformkörper (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 2,00 bis 3,00 m, bevorzugt 2,50 bis 3,00 m) entweder eine nur in geringerem Umfang (als im ersten Abschnitt) verdünnte Schüttung des wie beschrieben erhältlichen ringförmigen Vollkatalysators, oder, ganz besonders bevorzugt, eine alleinige (unverdünnte) Schüttung desselben ringförmigen Vollkatalysators, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysator von geringer, auf seinen Raumbedarf bezogener, Aktivmassendichte und im zweiten Abschnitt mit einem erfindungsgemäß erhältlichen ringförmigen Vollkatalysator mit hoher, auf seinen Raumbedarf bezogener, Aktivmassendichte beschickt werden (z.B. 6,5 mm x 3 mm x 4,5 mm [A x L x I] im ersten Abschnitt, und 5 x 2 x 2 mm im zweiten Abschnitt).

Insgesamt werden bei einer mit den wie beschrieben erhältlichen ringförmigen Vollkatalysatoren als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Butan, tert-Butanol bzw. dessen Methylether) von wenigstens 90 mol-%, oder 92 mol-%, vorzugsweise von wenigstens 95 mol-% resultiert. Die Selektivität der Acrolein- bzw. Methacroleinbildung wird dabei regelmäßig ≥ 94 mol-%, bzw. ≥ 95 mol-%, oder ≥ 96 mol-%, oder ≥ 97 mol-% betragen. In natürlicher Weise werden dabei möglichst geringe Heißpunkttemperaturen angestrebt.

Insgesamt bedingen dabei die wie beschrieben erhältlichen ringförmigen Vollkatalysatoren sowohl eine erhöhte Aktivität als auch eine erhöhte Selektivität der Zielproduktbildung.

Abschließend sei festgehalten, das die wie beschrieben erhältlichen ringförmigen Vollkatalysatoren auch ein vorteilhaftes Bruchverhalten bei der Reaktorbefüllung aufweisen. Auch ihr Druckverlustverhalten ist vorteilhaft. Im übrigen eignen sich die wie beschrieben erhältlichen ringförmigen Vollkatalysatoren ganz generell als Katalysatoren mit erhöhter Aktivität und Selektivität für gasphasenkatalytische Partialoxidationen organischer Verbindungen wie niederer (z.B. 3 bis 6 (d.h. 3, 4, 5, oder 6) C-Atome enthaltender) Alkane, Alkanole, Alkanale, Alkene und Alkenale zu olefinisch ungesättigten Adehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytisch oxidative Dehydrierungen organischer Verbindungen (z.B. 3, 4, 5, oder 6 C-Atome enthaltender).

Für das Verfahren der Propylenpartialoxidation zu Acrolein besonders vorteilhafte Stöchiometrien sind:
a) [Bi₂W₂O₉ x 2WO₃]_{0,5}[Mo₁₂Co_{5,5}Fe_{2,94}Si_{1,59}K_{0,08}Oₓ]₁;
b) Mo₁₂Ni_{6,5}Zn₂Fe₂Bi₁P_{0,0065}K_{0,06}Oₓ·10SiO₂;
c) Mo₁₂Co₇Fe_{2,94}Bi_{0,6}Si_{1,59}K_{0,08}Oₓ;
d) wie Multimetalloxid II-Vollkatalysator gemäß Beispiel 1 der DE-A 197 46 210; und
e) Wie Beispiel 1c aus der EP-A 015 565.

Der Wismutgehalt der wie beschrieben erhältlichen Aktivmassen kann auch wie in der DE-A 100 63 162 beschrieben eingestellt werden. Dabei wird aus Ausgangsverbindungen der elementaren Konstituenten der angestrebten Aktivmasse eine Lösung oder Suspension erzeugt, die zwar die zur Herstellung der Aktivmasse erforderliche Gesamtmenge der von Bi verschiedenen elementaren Konstituenten, aber nur eine Teilmenge des zur Herstellung der Aktivmasse erforderlichen Bi enthält, die Lösung oder Suspension unter Erhalt einer Trockenmasse getrocknet und die zur Herstellung der Aktivmasse zusätzlich benötigte Restmenge an Bi in Form einer Ausgangsverbindung des Bi wie in der DE-A 100 63 162 beschrieben unter Erhalt eines formbaren Gemischs in diese Trockenmasse eingearbeitet (z.B. wie im Beispiel der DE-A 100 63 162), das formbare Gemisch in erfindungsgemäßer Weise (d.h. nach Zugabe von Formungs- und gegebenenfalls Verstärkungshilfsmitteln) zu einem ringförmigen Vollkatalysatorformkörper geformt und dieser durch thermisches Behandeln (z.B. wie im Beispiel der DE-A 100 63 162) in den gewünschten ringförmigen Vollkatalysator überführt. Die Stöchiometrien (insbesondere der Ausführungsbeispiele) und thermischen Behandlungsbedingungen dieser (vorgenannten) Schrift sind für die Propylenpartialoxidation zu Acrolein gleichfalls besonders geeignet. Dies gilt insbesondere für die Stöchiometrie Mo₁₂Bi_{1,0}Fe₃Co₇Si_{1,6}K_{0,08}.

Die Inbetriebnahme einer frischen Katalysatorbeschickung mit wie beschrieben erhältlichen ringförmigen Vollkatalysatoren kann wie in der DE-A 10337788 beschrieben erfolgen. In der Regel nehmen Aktivität und Selektivität der Zielproduktbildung anfänglich mit der Betriebsdauer der Katalysatorbeschickung zu. Diese Formierung kann dadurch beschleunigt werden, dass man sie bei im wesentlichen gleichbleibendem Umsatz unter erhöhter Belastung der Katalysatorbeschickung mit Reaktionsgasausgangsgemisch durchführt und nach weitgehend vollzogener Formierung die Belastung auf ihren Zielwert zurücknimmt.

Es überrascht, dass das Verhältnis R von scheinbarer Massendichte zu wahrer Massendichte p (so wie es in der EP-A 1340538 definiert ist) bei den wie beschrieben erhältlichen ringförmigen Vollkatalysatoren in der Regel > 0,55 beträgt. Meist beträgt R ≤ 0,9 bzw. ≤ 0,8 und ≥ 0,6 bzw. ≥ 0,65.

Dabei ist R = 1 / (1 + V · p).
V ist das Porengesamtvolumen.

Weiterhin umfasst die vorliegender Erfindung im besonderen ein Verfahren zur Herstellung von ringförmigen Katalysatorformkörpern mit gekrümmter und/oder nicht gekrümmter Stirnfläche der Ringe, deren Aktivmasse eine Stöchiometrie der allgemeinen Formel IV,

Mo₁₂PₐV_{b}X_{c}¹X_{d}²Xₑ³Sb_{f}Re_{g}SₕOₙ (IV),

aufweist, in der Variablen folgende Bedeutung haben:
- X¹ =: Kalium, Rubidium und/oder Cäsium,
- X² =: Kupfer und/oder Silber,
- X³ =: Cer, Bor, Zirkonium, Mangan und/oder Wismut,
- a =: 0,5 bis 3,

- b =: 0,01 bis 3,
- c =: 0,2 bis 3,
- d =: 0,01 bis 2,
- e =: 0 bis 2,
- f =: 0,01 bis 2,
- g =: 0 bis 1,
- h =: 0,001 bis 0,5 und
- n =: eine Zahl, die durch die Wertigkeit und Häufigkeit der von Sauerstoff verschiedenen Elemente in IV bestimmt wird,
und deren ringförmige Geometrie jener der bereits beschriebenen ringförmigen Katalysatorformkörper mit Aktivmassen einer Stöchiometrie der allgemeinen Formel (I), (II), oder (III) entspricht.

Bevorzugt sind Aktivmassen IV, in denen h 0,03 bis 0,5 ist.

Besonders bevorzugte Stöchiometrie der allgemeinen Formel IV sind jene der Ausführungsbeispiele B1 bis B15 aus der EP-A 467 144 und dies auch dann, wenn diese beispielhaften Aktivmassen kein K enthalten.

Vorgenannten EP-A 467 144 beschreibt auch die Herstellung solcher ringförmigen Katalysatorformkörper und ihre Verwendung als Katalysatoren für die heterogen katalysierte Gasphasenpartialoxidation von Methacrolein zu Methacrylsäure. Diese Beschreibungen sind auch im in der vorliegenden Anmeldung gegebenen Kontext zutreffend, sieht man davon ab, dass bei der Herstellung der ringförmigen Katalysatorformkörper erfindungsgemäß Bornitrid als Gleitmittel zu verwenden ist.

D.h., ringförmige Katalysatorformkörper mit Aktivmassen der allgemeinen Stöchiometrie IV können dadurch hergestellt werden, dass man als Ausgangsverbindungen geeignete Salze der sie konstituierenden elementaren Bestandteile, gegebenenfalls bei erhöhter Temperatur und unter Zusatz von Säuren oder Basen, in wässrigem Medium durch Lösen und/oder Suspendieren fein verteilt und, zur Vermeidung unerwünschter Oxidationsprozesse ggf. unter Inertgas, mischt, das Gemisch zur Trockene eindampft, der resultierenden, feinteilige Form aufweisenden oder in feinteilige Form überführten Trockenmasse das erfindungsgemäß erforderliche Bornitrid sowie gegebenenfalls sonstige der genannten Formungshilfsmittel und Verstärkungsmittel zusetzt, die dabei erhaltene feinteilige Masse zur gewünschten Ringgeometrie formt (verdichtet) und die dabei resultierenden Katalysatorvorläuferformkörper anschließend thermisch behandelt. Bevorzugt wird die thermische Behandlung bei Temperaturen von 180 bis 480°C, besonders bevorzugt bei Temperaturen von 250 bis 450°C durchgeführt. Die thermische Behandlung kann dabei unter den bereits beschriebenen Gasatmosphären erfolgen. Beispielhaft erwähnt seien nochmals strömende Luft, strömende Inertatmosphäre (z.B. N₂, oder CO₂, oder Edelgase) oder Vakuum. Die thermische Behandlung kann in mehreren Temperaturstufen und/oder in verschiedenen Atmosphären durchgeführt werden. So kann z.B. in einer ersten Stufe bei 200 bis 260°C in Luft, in einer zweiten Stufe bei 420 bis 460°C in Stickstoff und in einer dritten Stufe bei 350 bis 410°C wiederum in Luft thermisch behandelt werden. Im Regelfall ist strömende Luft die bevorzugte Atmosphäre für die thermische Behandlung.

Im übrigen gilt das bei der Herstellung von ringförmigen Katalysatorformkörpern der Aktivmassen (I), (II) und (III) Gesagte hier in entsprechender Weise, jedoch mit dem Unterschied, dass hier die erhöhten Seitendruckfestigkeiten für die ringförmigen Vollkatalysatorvorläuferformkörper bevorzugt werden.

D.h., z.B. bevorzugtes Trocknungsverfahren für die wässrige Lösung oder Suspension der Quellen der elementaren Konstituenten der gewünschten Aktivmasse ist die Sprühtrocknung. Das resultierende Sprühpulver mit einer üblichen Körnung von 20 bis 50 µm wird erfindungsgemäß vorteilhaft, und erfindungsgemäß zweckmäßig nach Zusatz von feinteiligem Bornitrid als Hilfsmittel, zwischenkompaktiert, um das Pulver zu vergröbern. Bevorzugt erfolgt die Zwischenkompaktierung hier auf Partikeldurchmesser von 100 bis 2000 µm, bevorzugt von 150 bis 1500 µm und besonders bevorzugt von 400 bis 1000 µm. Anschließend erfolgt auf der Grundlage des vergröberten Pulvers die eigentliche Formgebung, wobei bei Bedarf zuvor nochmals feinteiliges erfindungsgemäßes Bornitrid (sowie gegebenenfalls weiter Formungs- und/oder Verstärkungshilfsmittel) zugegeben werden kann. Das bei der Herstellung der ringförmigen Katalysatorformkörper der Aktivmassen (I), (II) und (III) zu den Seitendruckfestigkeiten Gesagte gilt hier analog.

Bei der beschriebenen Herstellungsweise von ringförmigen Katalysatorformkörpern aus Aktivmassen der allgemeinen Formel IV wird Antimon üblicherweise als Antimontrioxid, Rhenium z.B. als Rhenium(VII)oxid, Molybdän vorzugsweise als Ammoniumsalz der Molybdän- oder Phosphormolybdänsäure, Bor z.B. als Borsäure, Vanadin in der Regel als Ammoniumvanadat oder Vanadinoxalat, Phosphor mit Vorteil als orthoPhosphorsäure oder Di-Ammonium-Phosphat, Schwefel z.B. als Ammoniumsulfat und die kationischen Metalle normalerweise als Nitrate, Oxide, Hydroxide, Carbonate, Chloride, Formiate, Oxalate und/oder Acetate bzw. deren Hydrate eingesetzt. Bevorzugte Ringgeometrie des fertiggestellten Katalysatorformkörpers ist hier die Geometrie 7mm x 7mm x 3mm (Außendurchmesser x Länge x Innendurchmesser). Die katalytische Gasphasenoxidation von Methacrolein zu Methacrylsäure unter Anwendung der wie beschrieben erhältlichen ringförmigen Katalysatorformkörper kann in an sich bekannter, z.B. in der EP-A 467 144 beschriebener, Weise erfolgen. Das Oxidationsmittel Sauerstoff kann z.B. in Form Luft, aber auch in reiner Form eingesetzt werden. Aufgrund der hohen Reaktionswärme werden die Reaktionspartner vorzugsweise mit Inertgasen wie N₂, CO, CO₂ und/oder mit Wasserdampf verdünnt. Vorzugsweise wird bei einem Methacrolein : Sauerstoff : Wasserdampf : Inertgas Verhältnis von 1 : (1 bis 3) : (2 bis 20) : (3 bis 30), besonders bevorzugt von 1 : (1bis 3) : (3 bis 10) : (7 bis 18) gearbeitet. Der Anteil des Methacrolein am Reaktionsgasausgangsgemisch beläuft sich dabei in der Regel auf 4 bis 11, vielfach 4,5 bis 9 Vol.-%. Der Sauerstoffgehalt wird zur Vermeidung explosiver Gemische bevorzugt auf ≤ 12,5 Vol.-% beschränkt. Dies wird besonders bevorzugt durch Rückführung eines Teilstroms des vom Reaktionsprodukt abgetrennten Abgases erreicht. Im übrigen erfolgt die Gasphasenpartialoxidation zur Methacrylsäure in typischer Weise bei Gesamtraumbelastungen des Katalysatorfestbetts von 800 bis 1800 Nl/l·h, bzw. bei Methacroleinbelastungen von 60 bis 140 Nl/l·h. Als Reaktor wird in der Regel ein Rohrbündelreaktor eingesetzt. Reaktionsgas und Salzbad können dabei über den Reaktor betrachtet sowohl im Gleichstrom als auch im Gegenstrom geführt werden. Das Salzbad selbst wird normalerweise in mäandrierender Form durch den Reaktor geführt. Bevorzugtes Bornitrid zur Herstellung von ringförmigen Katalysatorformkörpern aus Aktivmassen der allgemeinen Stöchiometrie IV ist ebenfalls Boron Nitride Grade A 01, Number PD-5006, Issue 0.-07.99 der Firma H.C. Starck.

Das erfindungsgemäße Verfahren umfasst weiterhin insbesondere ein Verfahren zur Herstellung von ringförmigen Katalysatorformkörpern mit gekrümmter und/oder nicht gekrümmter Stirnfläche der Ringe, deren Aktivmasse ein Vandium, Phosphor und Sauerstoff enthaltendes Multimetalloxid ist, und die sich als Katalysatoren für die heterogenkatalytische Gasphasenoxidation wenigstens eines Kohlenwasserstoffs mit mindestens vier Kohlenstoffatomen (insbesondere n-Butan, n-Butene und/oder Benzol) zu Maleinsäureanhydrid eignen. Die Herstellung entsprechender ringförmiger Katalysatorformkörper ist in der WO 03/078310 unter Zusatz von Graphit als Formungshilfsmittel beschrieben. Alle Ausführungen der WO 03/078310 und alle in der WO 03/078310 angesprochenen Katalysatoren besitzen auch dann Tragfähigkeit und die in der WO 03/078310 angesprochene Anwendbarkeit, wenn die in der WO 03/078310 offenbarten Herstellmaßnahmen beibehalten werden, und das im Rahmen der Herstellung mitverwendete Graphit erfindungsgemäß durch gewichtsidentische Mengen an Bornitrid ersetzt wird. Erfindungsgemäß bevorzugt wird auch hier Boron Nitride Grade A 01, Number PD-5006, Issue 0-07.99 der Firma H.C. Starck als Graphitersatz angewendet. Auch im vorgenannten Fall der Vanadium, Phosphor und Sauerstoff enthaltenden ringförmigen Multimetalloxidkatalysatoren stellen sich die erfindungsgemäßen Vorteile ein. Dies gilt im Besonderen für alle Ausführungsbeispiele der WO 03/078310.

Das erfindungsgemäße Verfahren umfasst weiterhin insbesondere Verfahren zur Herstellung von erfindungsgemäßen, z.B. kugelförmigen, vollzylinderförmigen oder ringförmigen Katalysatorformkörpern mit gekrümmter und/oder nicht gekrümmter Stirnfläche der Ringe, deren Aktivmasse ein Mo, V und wenigstens eines der Elemente Te und Sb enthaltendes Multimetalloxid ist, wie es z.B. die Schriften EP-A 962 253, DE-A 101 22 027, EP-A 608 838, DE-A 198 35 247, EP-A 895 809, EP-A 1 254 709, EP-A 1 192 987, EP-A 1 262 235, EP-A 1 193 240, JP-A 11-343261, JP-A 11-343262, EP-A 1 090 684, EP-A 1 301 457, EP-A 1 254 707, EP-A 1 335 793, DE-A 100 46 672, DE-A 100 34 825, EP-A 1 556 337, DE-A 100 33 121, WO 01/98246, EP-A 1 558 569 beschrieben.

Häufig enthalten die vorgenannten Multimetalloxidmassen noch das Element Nb. Die vorgenannten Multimetalloxidkatalysatoren eignen sich bei erfindungsgemäßer Herstellung für alle in den vorgenannten Schriften durchgeführten katalysierten Gasphasenreaktionen. Dies sind im besonderen die heterogen katalysierte partielle Gasphasenoxidation von Propan zu Acrylsäure sowie von Acrolein zu Acrylsäure, von Methacrolein zu Methacrylsäure und von iso-Butan zu Methacrylsäure.

Abschließend sei an dieser Stelle noch festgehalten, dass erfindungsgemäß hergestellte Katalysatorformkörper nicht in notwendiger Weise als solche als Katalysatoren für heterogen katalysierte Gasphasenreaktionen eingesetzt werden müssen. Vielmehr können sie einer Mahlung unterworfen werden und nach Klassieren des dabei resultierenden feinteiligen Materials mit Hilfe eines geeigneten flüssigen Bindemittels auf die Oberfläche eines geeigneten Trägerkörpers aufgebracht werden. Nach Trocknung oder unmittelbar nach Auftragung der Aktivmassenschale auf den Trägerkörper kann der resultierende Schalenkatalysator als Katalysator für heterogen katalysierte Gasphasenreaktionen eingesetzt werden, wie es z.B. die DE-A 101 22 027 beschreibt.

Zusammenfassend sei nochmals festgehalten, dass die erfindungsgemäß erhältlichen Katalysatorformkörper in hervorragender Weise als Katalysatoren für heterogen katalysierte Reaktionen in der Gasphase geeignet sind. Zu diesen Gasphasenreaktionen zählen vor allem die partiellen Oxidationen organischer Verbindungen, die partiellen Ammoxidationen organischer Verbindungen sowie die Oxidehydrierungen organischer Verbindungen. Als partielle heterogen katalysierte Oxidationen organischer Verbindungen kommen vor allem die in der DE-A 10 2004 025 445 genannten in Betracht. Beispielhaft seien nochmals genannt die Umsetzung von Propylen zu Acrolein und/oder Acrylsäure (vgl. z.B. DE-A 23 51 151), die Umsetzung von tert-Butanol, iso-Buten, iso-Butan, iso-Butyraldehyd oder dem Methylether des tert.-Butanols zu Methacrolein und/oder Methacrylsäure (vgl. z.B. DE-A 25 26 238, EP-A 092 097, EP-A 58927, DE-A 4132263, DE-A 4132684 und DE-A 4022212), die Umsetzung von Acrolein zu Acrylsäure, die Umsetzung von Methacrolein zu Methacrylsäure (vgl. z.B. die DE-A 2526238), die Umsetzung von o-Xylol, p-Xylol oder Naphtalin zu Phthalsäureanhydrid (vgl. z.B. EP-A 522 871) oder den entsprechenden Säuren sowie die Umsetzung von Butadien zu Maleinsäureanhydrid (vgl. z.B. DE-A 21 06 796 und DE-A 16 24 921), die Umsetzung von n-Butan zu Maleinsäureanhydrid (vgl. z.B. GB-A 1 464 198 und GB-A 1 291 354), die Umsetzung von Indenen zu z.B. Anthrachinon (vgl. z.B. DE-A 20 25 430), die Umsetzung von Ethylen zu Ethylenoxid oder von Propylen zu Propylenoxid (vgl. z.B. DE-AS 12 54 137, DE-A 21 59 346, EP-A 372 972, WO 89/07101, DE-A 43 11 608 und Beyer, Lehrbuch der organischen Chemie, 17. Auflage (1973), Hirzel Verlag, Stuttgart, S. 261), die Umsetzung von Propylen und/oder Acrolein zu Acrylnitril (vgl. z.B. DE-A 23 51 151), die Umsetzung von iso-Buten und/oder Methacrolein zu Methacrylnitril (d.h., der Begriff der partiellen Oxidation soll in dieser Schrift auch die partielle Ammoxidation, d.h., eine partielle Oxidation im Beisein von Ammoniak umfassen), die oxidative Dehydrierung von Kohlenwasserstoffen (vgl. z.B. DE-A 23 51 151), die Umsetzung von Propan zu Acrylnitril oder zu Acrolein und/oder Acrylsäure (vgl. z.B. DE-A 101 31 297, EP-A 1 090 684, EP-A 608 838, DE-A 100 46 672, EP-A 529 853, WO 01/96270 und DE-A 100 28 582), die Umsatzung von iso-Butan zu Methacrolein und/oder Methacrylsäure, so wie die Reaktionen von Ethan zu Essigsäure, von Ethylen zu Ethylenoxid, von Benzol zu Phenol sowie von 1-Buten oder 2-Buten zu den entsprechenden Butandiolen.

Selbstverständlich kann es sich bei der Gasphasenreaktion aber auch um eine heterogen katalysierte Hydrierung oder um eine heterogen katalysierte Dehydrierung organischer Verbindungen handeln. Es besticht insbesondere die Langzeitstabilität der Katalysatoren, selbst dann, wenn bei den Reaktionen, z.B. in Rohrbündelreaktoren, Heißpunkte (maximale Reaktionstemperaturen) auftreten, die den bei der erfindungsgemäßen Katalysatorherstellung im Rahmen der thermischen Behandlung angewendeten Temperaturen im wesentlichen entsprechen.

Ganz generell führt das erfindungsgemäße Verfahren zu Multielementoxidkatalysatoren, die, bezogen auf die Multielementoxidmasse 0,1 bis 20 Gew.-% bzw. bis 10 Gew.%, oder 0,3 bis 8 Gew.-%, vielfach 0,5 bis 6 Gew.-% bzw. 0,5 bis 5 Gew.-% an Bornitrid enthalten. Letzteres ist im Röntgendiffraktogramm des Multielementoxidkatalysators gut nachweisbar.

Beispiele und Vergleichsbeispiele (das verwendete Gleithilfsmittel bleibt im Rahmen der Herstellung stets erhalten, wird infolge seiner Inertheit aber nicht als Bestandteil der-Aktivmasse aufgeführt)

### 1. Vergleichsbeispiele für eine heterogen katalysierte Partialoxdiation von Propen zu Acrolein

### A) Herstellung von ringförmigen Vollkatalysatoren mit der nachfolgenden Stöchiometrie S1 der Aktivmasse: Mo₁₂Co₇Fe_{2,94}Bi_{0,6}Si_{1,59}K_{0,8}Oₓ.

Bei 60°C wurden 213 kg Ammoniumheptamolybdattetrahydrat (81,5 Gew.-% MoO₃) in 600 I Wasser gelöst. In diese Lösung wurden unter Aufrechterhaltung der 60°C 0,97 kg einer 46,8 gew.-%igen wässrigen Kaliumhydroxidlösung von 20°C eingerührt (dabei wurde eine Lösung A erhalten).

Eine zweite Lösung B wurde hergestellt, indem man unter Rühren zu 333,7 kg einer wässrigen Kobalt-(II)-nitratlösung (12,4 Gew.-% Co) bei 30°C 116,25 kg einer 20°C aufweisenden wässrigen Eisen-(III)-nitratlösung (14,2 Gew.-% Fe) gab. Nach beendeter Zugabe wurde noch 30 min. bei 30°C gerührt. Danach wurden bei 60°C 112,3 kg einer 20°C aufweisenden wässrigen Wismutnitratlösung (11,2 Gew.-% Bi) unter Erhalt der Lösung B eingerührt. Innerhalb von 30 min. wurde bei 60°C die Lösung B in die Lösung A eingerührt. 15 min. nach beendetem Einrühren wurden bei 60°C 19,16 kg Kieselsol (der Fa. Du Pont, Typ Ludox^{®}, 46,80 Gew.-% SiO₂, Dichte: 1,36 bis 1,42 g/cm³, pH = 8,5 bis 9,5, Alkaligehalt max. 0,5 Gew.-%) in die erhaltene Maische gegeben. Unter Aufrechterhaltung der 60°C wurde noch 15 min. nachgerührt. Dann wurde die erhaltene Maische im Gegenstromverfahren sprühgetrocknet (Gaseingangstemperatur: 400 ± 10°C, Gasausgangstemperatur: 140 ± 5°C) wobei ein Sprühpulver erhalten wurde, dessen Glühverlust (3 h bei 600°C unter Luft) 30 % seines Gewichtes betrug. Die Körnung des Sprühpulvers betrug im wesentlichen einheitlich 30 µm.

In Teilmengen des erhaltenen Sprühpulvers wurden jeweils zusätzlich 1,5 Gew.-% (bezogen auf die Sprühpulvermenge) feinteiliges synthetisches Graphit vom Typ TIMREX T44, der Firma Timcal AG (CH-Bodio) eingemischt. Es weist folgende Reinheit und sonstige Eigenschaften auf:

| | |
|---|---|
| Ascherückstand (Glühen bei 815°C unter Luft) | 0,07 Gew.-% |
| Feuchtigkeit (unter Normalbedingungen) | 0,1 Gew.-% |
| Al | 15 Gew.ppm |
| As | < 0,5 Gew.ppm |
| Ca | 100 Gew.ppm |
| Co | < 1 Gew.ppm |
| Cr | < 1 Gew.ppm |
| Cu | < 1 Gew.ppm |
| Fe | 60 Gew.ppm |
| Mo | < 1 Gew.ppm |
| Ni | 2 Gew.ppm |
| Pb | < 2 Gew.ppm |
| Sb | < 0.1 Gew.ppm |
| Si | 80 Gew.ppm |
| Ti | 150 Gew.ppm |
| V | 17 Gew.ppm |
| S | 60 Gew.ppm |

Spezifische BET-Oberfläche: 6 bis 13 m²/g, typisch 10 m²/g

| | |
|---|---|
| Typische Teilchengrößenverteilung (Laser Malvern) | D10: 4,8 µm |
| | D50: 19,3 µm |
| | D90: 44,7 µm |
| Schwankungsbreite | D90: 37 bis 52 µm |

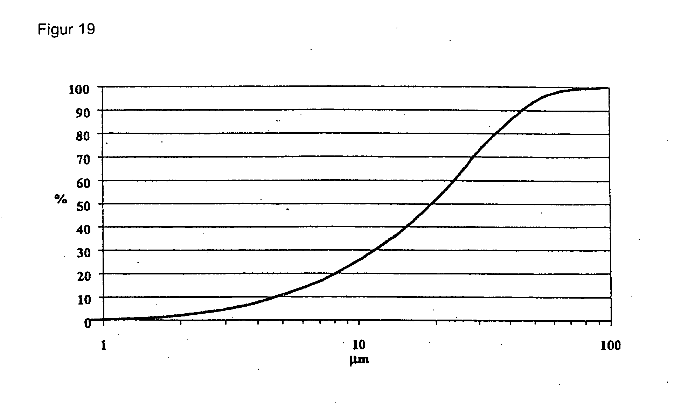

Die Gesamtteilchengrößenverteilung zeigt Figur 19. Dabei zeigt die Abszisse die Durchmesser im logarithmischen Maßstab. Die Ordinate zeigt den Prozentanteil der Teilchenzahl mit dem jeweiligen Durchmesser.

| | |
|---|---|
| Crystallite Height | min. 100 nm |
| Interlayer Distance | 0,3354-0,3359 nm |

Das dabei jeweils resultierende Trockengemisch wurde mittels eines Kompaktors der Fa. Hosokawa Bepex GmbH (D-74211 Leingarten) vom Typ Kompaktor K 200/100 unter den Bedingungen von 2,8 mm Spaltbreite, 1,0 mm Siebweite, 400 µm Siebweite Unterkorn, 60 kN Presssollkraft und 65 bis 70 Upm Schneckendrehzahl durch Vorkompaktieren auf eine im wesentlichen einheitliche Korngröße von 400 µm bis 1 mm vergröbert. Das Kompaktat hatte eine Härte von 10 N.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ Rx73, der Fa. Kilian, D-50735 Köln, unter Stickstoffatmosphäre zu ringförmigen Vollkatalysatorvorläuferformkörpern mit nicht gekrümmter Stirnfläche der Geometrie 5 mm x 3 mm x 2 mm (A x L x I) mit unterschiedlicher Seitendruckfestigkeit verdichtet.

Die resultierenden Vollkatalysatorvorläuferformkörper und ihre Seitendruckfestigkeiten waren:
VVV 1: 15 N;
VVV 2: 20 N.

Zur abschließenden thermischen Behandlung wurden jeweils 1900 g der Vollkatalysatorvorläuferformkörper in einer beheizbaren Umluftkammer (0,12 m³ Innenvolumen) aufgeschüttet (2 Nm³ Luft/min.). Anschließen wurde die Temperatur in der Schüttung wie folgt verändert:
- mit 1°C/min. von 25°C auf 160°C erhöht;
- dann 100 min. bei 160°C gehalten;
- danach mit 3°C/min. von 160°C auf 200°C erhöht;
- dann 100 min. bei 200°C gehalten;
- danach mit 2°C/min. von 200°C auf 230°C erhöht;
- dann 100 min. bei 230°C gehalten;
- danach mit 3°C/min. von 230°C auf 270°C erhöht;
- dann 100 min. bei 270°C gehalten;
- danach mit 1°C/min. auf 380°C erhöht;
- dann 4,5 h bei 380°C gehalten;
- danach mit 1°C/min. auf 430°C erhöht;
- dann 4,5 h bei 430°C gehalten;
- danach mit 1°C/min, auf 500°C erhöht;
- dann 9 h bei 500°C gehalten;
- danach innerhalb von 4 h auf 25°C abgekühlt.

Dabei wurden aus den ringförmigen Vollkatalysatorvorläuferformkörpern die nachfolgenden ringförmigen Vollkatalysatoren erhalten (der erste Buchstabe V steht für Vergleichsbeispiel):

VVV 1 → VVK 1;

VVV 2 → VVK 2.

Die Parameter O, V, der wesentliche, den größten Beitrag zum Porengesamtvolumen leistende, Porendurchmesser d^{max} sowie der prozentuale Anteil derjenigen Porendurchmesser am Porengesamtvolumen, deren Durchmesser > 0,1 und < 1 µm betragen, waren von diesen ringförmigen Vollkatalysatoren wie folgt beschaffen:
VVK 1: O = 6,4 m²/g; V = 0,32 cm³/g; d^{max} = 0,32 µm; V^{0,1}₁₋% = 91%.
VVK 2: O = 6,8 m²/g; V = 0,34 cm³/g; d^{max} = 0,36 µm; V^{0,1}₁₋% = 87%.

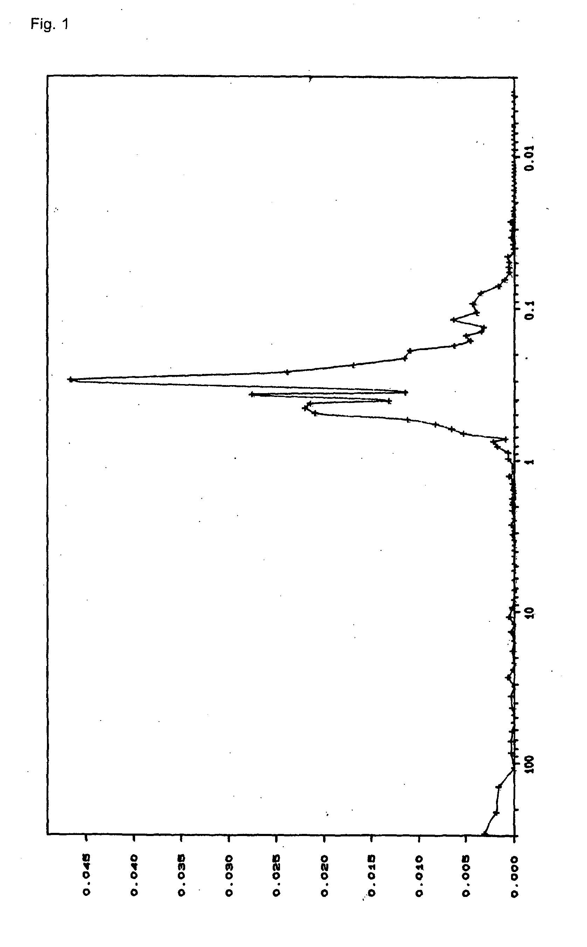

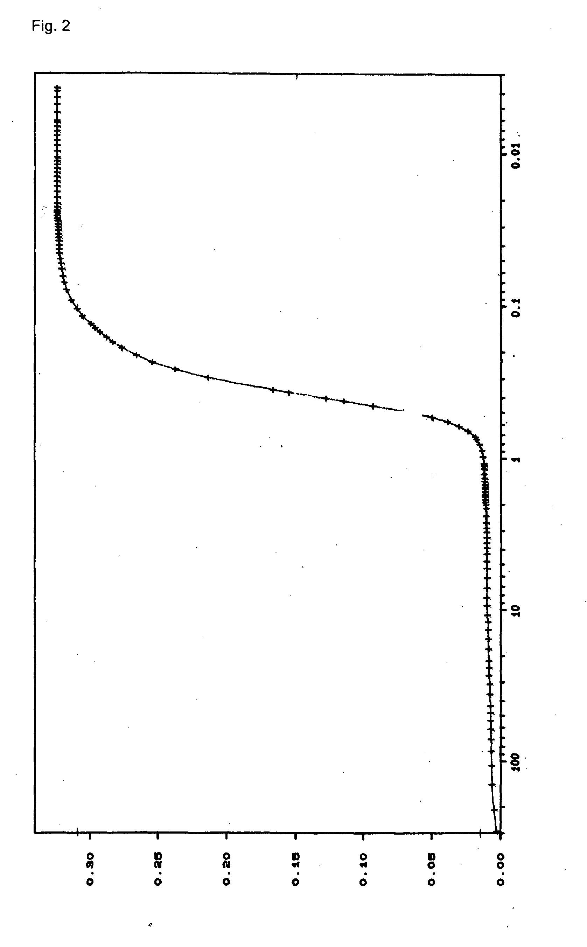

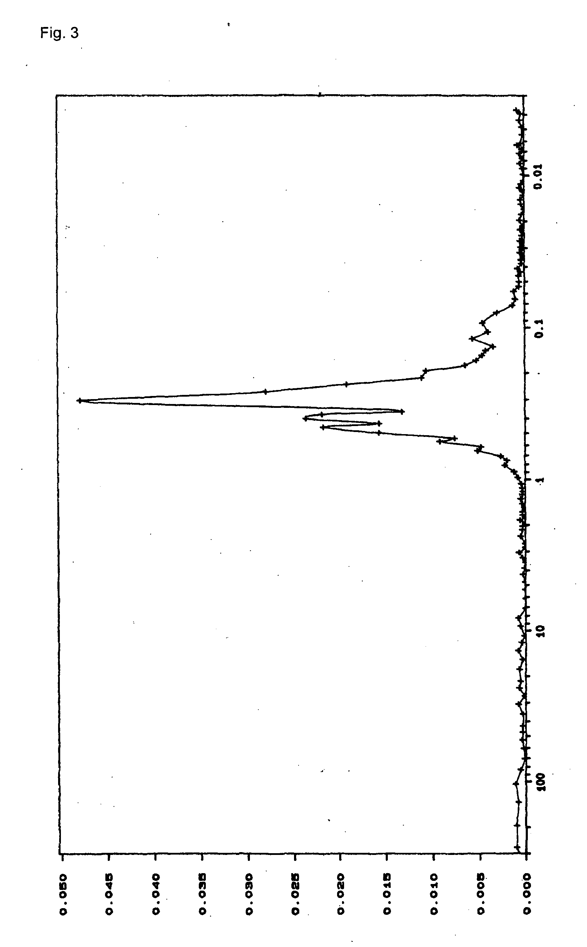

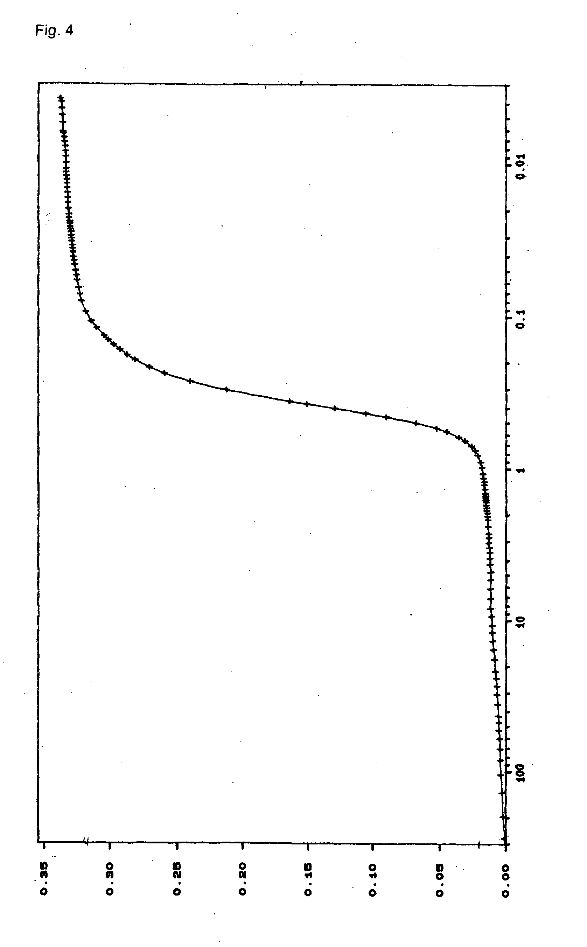

Die Figuren 1 (3) und 2 (4) zeigen außerdem die Porenverteilung des ringförmigen Vollkatalysators WK1 (VVK2). In Fig. 1 (3) zeigt die Abszisse den Porendurchmesser in µm und die Ordinate den differentiellen Beitrag in ml/g des jeweiligen Porendurchmessers zum Porengesamtvolumen. In Fig. 2(4) zeigt die Abszisse ebenfalls den Porendurchmesser in µm und die Ordinate das Integral über die individuellen Beiträge der einzelnen Porendurchmesser zum Porengesamtvolumen in ml/g.

(Anstatt die thermische Behandlung wie beschrieben durchzuführen, kann man sie auch wie in Beispiel 3 der DE-A 10046957 beschrieben mittels einer Bandcalziniervorrichtung durchführen; die Kammern besitzen eine Grundfläche (bei einer einheitlichen Kammerlänge von 1,40 m) von 1,29 m² (Zersetzung, Kammern 1-4) und 1,40 m² (Calcination, Kammern 5-8) und werden von unten durch das grobmaschige Band von 70-120 Nm³/h Zuluft, bevorzugt von 75 Nm³/h Zuluft, durchströmt, die mittels rotierender Ventilatoren angesaugt werden; innerhalb der Kammern war die zeitliche und örtliche Abweichung der Temperatur vom Sollwert stets ≤ 2°C; durch die Kammern werden die ringförmigen Vollkatalysatorvorläuferformkörper in einer Schichthöhe von 50 mm bis 110 mm, bevorzugt von 50 mm bis 70 mm, geführt; im übrigen wird wie in Beispiel 3 der DE-A 100 46 957 beschrieben verfahren; die resultierenden ringförmigen Vollkatalysatoren können wie die ringförmigen Vollkatalysatoren VVK1 und VVK2 für die nachfolgend unter C) beschriebene gasphasenkatalytische Partialoxidation von Propen zu Acrolein eingesetzt werden).

### B) Herstellung von ringförmigen Vollkatalysatoren mit der nachfolgenden Stöchiometrie S2 der Aktivmasse:

[Bi₂W₂O₉ • 2WO₃]_{0,5}[Mo₁₂CO_{5,5}Fe_{2,94}Si_{1,59}K_{0,08}Oₓ]₁.

### 1. Herstellung einer Ausgangsmasse 1

In 775 kg einer wässrigen salpetersauren Wismutnitratlösung (11,2 Gew.-% Bi; freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wässrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver (Partikelgröße im wesentlichen einheitlich 30 µm), das einen Glühverlust von 12 Gew.-% aufwies (3 h bei 600°C unter Luft glühen), wurde anschließend mit 16,8 Gew.-% (bezogen auf das Pulver) Wasser in einem Kneter angeteigt und mittels eines Extruders (Drehmoment: ≤ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min bei Temperaturen von 90-95°C (Zone 1), 115°C (Zone 2) und 125°C (Zone 3) an Luft getrocknet und dann bei einer Temperatur im Bereich von 780 bis 810°C thermisch behandelt (calciniert; im luftdurchströmten Drehrohrofen (0,3 mbar Unterdruck, 1,54 m³ Innenvolumen, 200 Nm³ Luft/h)). Wesentlich bei der genauen Einstellung der Calcinationstemperatur ist, dass sie an der angestrebten Phasenzusammensetzung des Calcinationsprodukts orientiert zu erfolgen hat. Gewünscht sind die Phasen WO₃ (monoklin) und Bi₂W₂O₉, unerwünscht ist das Vorhandensein von Y-Bi₂WO₆ (Russellit). Sollte daher nach der Calcination die Verbindung γ-Bi₂WO₆ anhand eines Reflexes im Pulverröntgendiffraktogramm bei einem Reflexwinkel von 2⊝ = 28,4° (CuKα-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde gemahlen, so dass der X₅₀-Wert (vgl. Ullmann's Encyclopedia of Industrial Chemistry, 6^{th} Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem SiO₂ der Fa. Degussa vom Typ Sipernat^{®} (Rüttelgewicht 150 g/l; X₅₀-Wert der SiO₂-Partikel betrug 10 µm, die BET-Oberfläche betrug 100 m²/g) vermischt. Alternativ können auch nur 0,5 Gew.-% Sipernat eingesetzt werden.

### 2. Herstellung einer Ausgangsmasse 2

Eine Lösung A wurde hergestellt, indem man bei 60°C unter Rühren in 600 I Wasser 213 kg Ammoniumheptamolybdattetrahydrat (81,5 Gew.-% MoO₃) löste und die resultierende Lösung unter Aufrechterhaltung der 60°C und Rühren mit 0,97 kg einer 20°C aufweisenden wässrigen Kaliumhydroxidlösung (46,8 Gew.-% KOH) versetzte.

Eine Lösung B wurde hergestellt indem man bei 60°C in 262,9 kg einer wässrigen Co-(II)-baltnitratlösung (12,4 Gew.-% Co) 116,25 kg einer wässrigen Eisen-(III)-nitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wässrigen Gemisch 19,16 kg eines Kieselgels der Fa. Du Pont vom Typ Ludox (46,80 Gew.-% SiO₂, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintriftstemperatur: 400 ± 10°C, Gasaustrittstemperatur: 140 ± 5°C). Das resultierende Sprühpulver wies einen Glühverlust von ca. 30 Gew.-% (3 h bei 600°C unter Luft glühen) und eine im wesentlichen einheitliche Körnung von 30 µm auf.

### 3. Herstellung der Multimetalloxidaktivmasse

Die Ausgangsmasse 1 wurde mit der Ausgangsmasse 2 in den für eine Multimetalloxidaktivmasse der Stöchiometrie

[Bi₂W₂O₉ · 2WO₃]_{0,5}[MO₁₂Co_{5,5}Fe_{2,94}Si_{1,59}K_{0,08}Oₓ]₁

erforderlichen Mengen in einem Mischer mit Messerköpfen homogen vermischt. Bezogen auf die vorgenannte Gesamtmasse wurden zusätzlich 1 Gew.-% des bereits erwähnten feinteiligen Graphit der Fa. Timcal AG (CH-Bodio), vom Typ TIMREX T44 homogen eingemischt. Das resultierende Gemisch wurden dann in einem Kompaktor (Fa. Hosokawa Bepex GmbH, D-74211 Leingarten) vom Typ Kompaktor K200/100 mit konkaven, geriffelten Glattwalzen gefahren (Spaltweite: 2,8 mm, Siebweite: 1,0 mm, Siebweite Unterkorn: 400 µm, Presssollkraft: 60 kN, Schneckendrehzahl: 65 bis 70 Umdrehungen je Minute). Das resultierende Kompaktat wies eine Härte von 10 N und eine im wesentlichen einheitliche Körnung von 400 µm bis 1 mm auf.
Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ R x 73, der Fa. Kilian, D-50735 Köln, unter Stickstoffatmosphäre zu ringförmigen Vollkatalysatorvorläuferformkörpern unterschiedlicher Geometrie (A x L x I) mit unterschiedlicher Seitendruckfestigkeit verdichtet.

Die resultierenden Vollkatalysatorvorläuferformkörper, ihre Geometrien und ihre Seitendruckfestigkeiten waren:

| | | |
|---|---|---|
| VVV3: | 5 mm x 3 mm x 2 mm; | 19 N (Masse: 129 mg). |
| VVV4: | 5mmx3mmx3mm; | 16 N. |
| VVV5: | 5mmx3mmx3mm; | 17 N. |
| VVV6: | 5,5 mm x 3 mm x 3,5 mm; | 14 N. |
| VVV7: | 5,5 mm x 3 mm x 3,5 mm; | 15,5 N. |
| VVV8: | 6 mm x 3 mm x 4 mm; | 13 N. |
| VVV9: | 6 mm x 3 mm x 4 mm; | 16,3 N. |
| VVV10: | 6,5 mm x 3 mm x 4,5 mm; | 15,6 N. |
| VVV11: | 7 mm x 3 mm x 5 mm; | 16,3 N. |

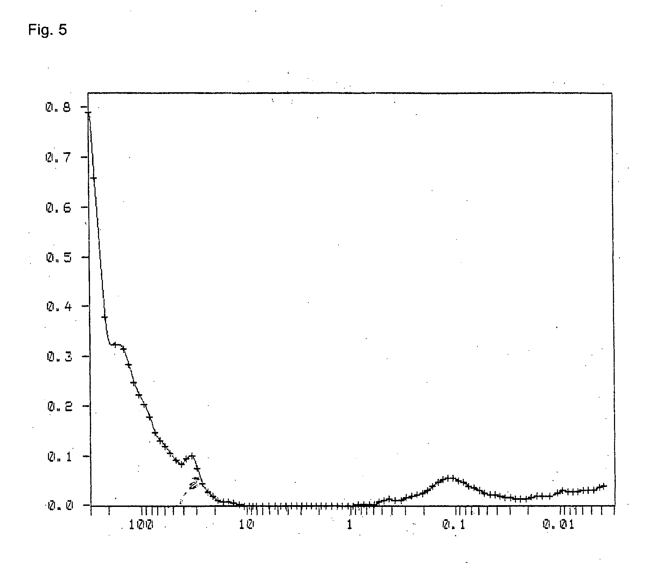

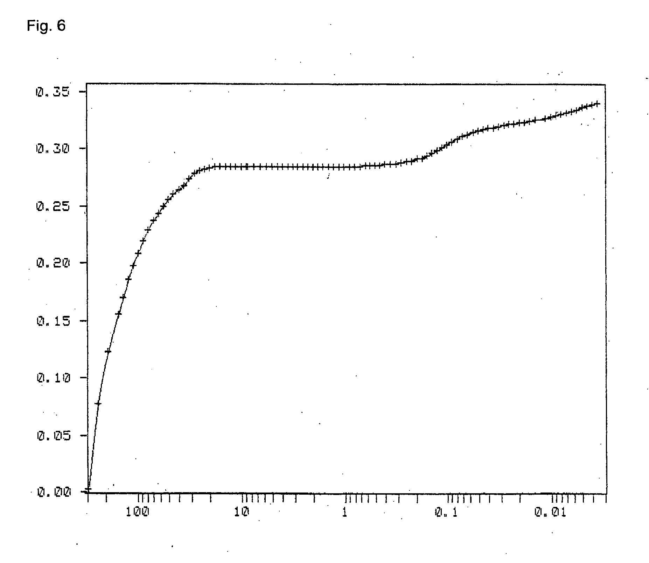

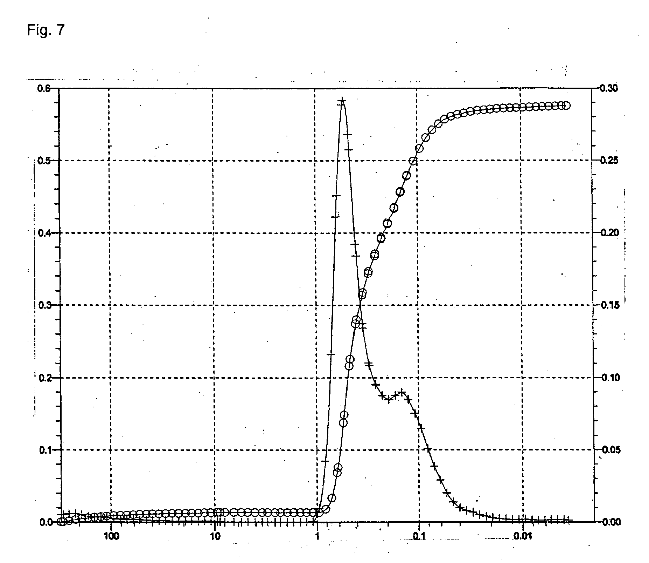

Fig. 5 (6) zeigt die Porenverteilung im ringförmigen Vollkatalysatorvorläuferformkörper VVV3. Die Achsenbeschriftung von Fig. 5 entspricht derjenigen von Fig. 7 und die Achsenbeschriftung von Fig. 6 entspricht derjenigen von Fig. 2.

Zur abschließenden thermischen Behandlung wurden jeweils 1000 g der Vollkatalysatorvorläuferformkörper in einem Luft durchströmten Muffelofen (60 I Innenvolumen, 1 l/h Luft pro Gramm Vollkatalysatorvorläuferformkörper) zunächst mit einer Aufheizrate von 180°C/h von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten, bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.

Dabei wurden aus den ringförmigen Vollkatalysatorvorläuferformkörpern die nachfolgenden ringförmigen Vollkatalysatoren erhalten (der erste Buchstabe V steht jeweils für Vergleichsbeispiel):

| | O [m²/g] | V [cm³/g] | d^{max} [µm] | V^{0,1}₁-% | R |
|---|---|---|---|---|---|
| VVV3 → VVK3 | 7,6 | 0,27 | 0,6 | 79 | 0,66 |
| VVV4 → VVK4 | 6,9 | 0,23 | 0,45 | 70 | - |
| VVV5 → VVK5 | - | - | - | - | - |
| VVV6 → VVK6 | 7,45 | 0,21 | 0,40 | 74 | - |
| VVV7 → VVK7 | 7,95 | 0,205 | 0,39 | 73 | 0,68 |
| VVV8 → VVK8 | 7,6 | 0,22 | 0,45 | 74 | - |
| VVV9 → VVK9 | 9,61 | 0,22 | 0,30 | 70 | 0,68 |
| VVV10 → VVK10 | - | - | - | - | - |
| VVV11 → VVK11 | - | - | - | - | - |

Zusätzlich enthält die vorstehende Tabelle Werte für die spezifische Oberfläche O, das Porengesamtvolumen V, den Porendurchmesser d^{max} der den größten Beitrag zum Porengesamtvolumen leistet, sowie die prozentualen Anteile derjenigen Porendurchmesser am Porengesamtvolumen, deren Durchmesser > 0,1 und < 1 µm betragen und R-Werte.

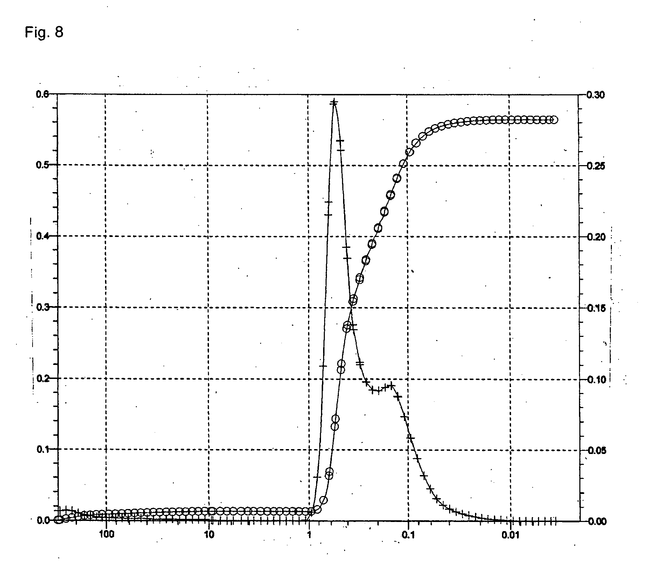

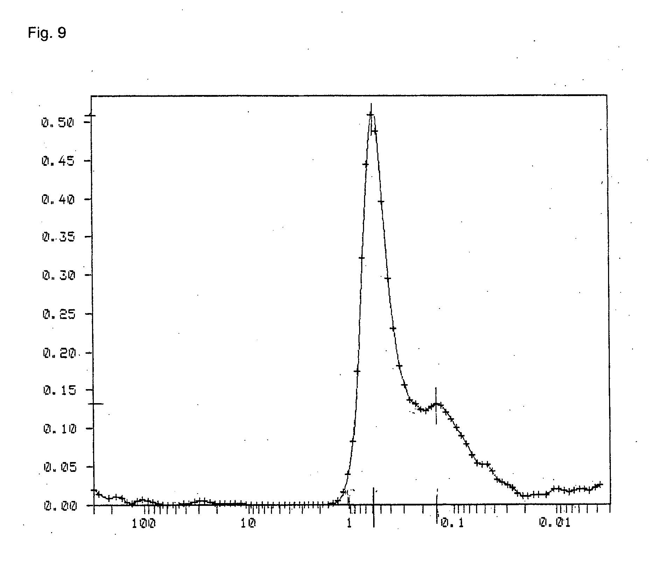

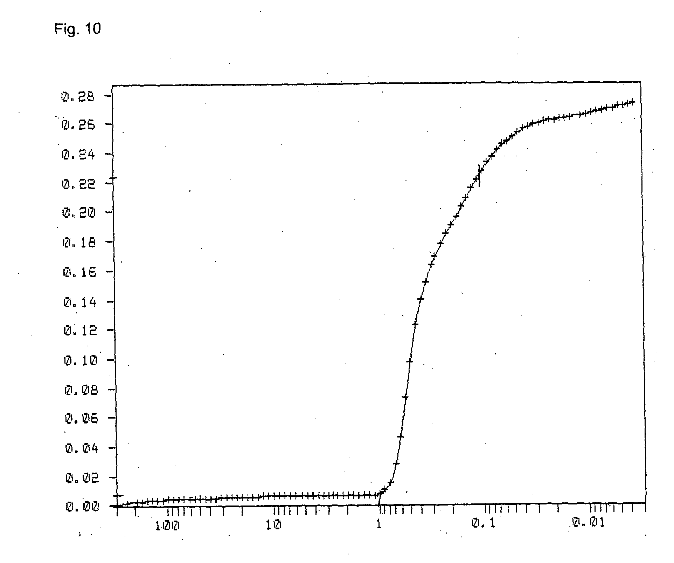

Die Figuren 7 und 8 zeigen außerdem die Porenverteilung des ringförmigen Vollkatalysators VVK3 für zwei voneinander unabhängige Reproduktionen. Auf der Abszisse ist der Porendurchmesser in µm aufgetragen. Auf der linken Ordinate ist der Logarithmus des differentiellen Beitrags in ml/g des jeweiligen Porendurchmessers zum Porengesamtvolumen aufgetragen (Kurve +). Das Maximum weist den Porendurchmesser mit dem größten Beitrag zum Porengesamtvolumen aus. Auf der rechten Ordinate ist in ml/g das Integral über die individuellen Beiträge der einzelnen Porendurchmesser zum Porengesamtvolumen aufgetragen (Kurve O). Der Endpunkt ist das Porengesamtvolumen. Die Figuren 9 und 10 zeigen die Porenverteilung einer weiteren Reproduktion des VVK3 bei gleicher Achsenbeschriftung wie in Fig. 7, 8.

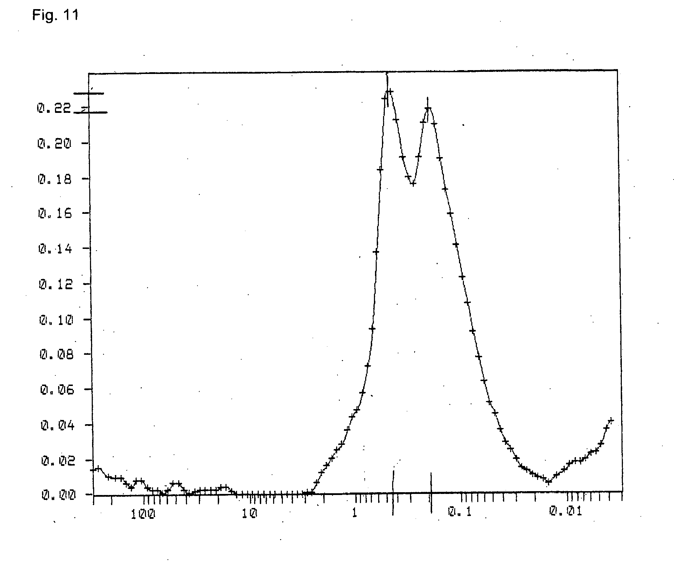

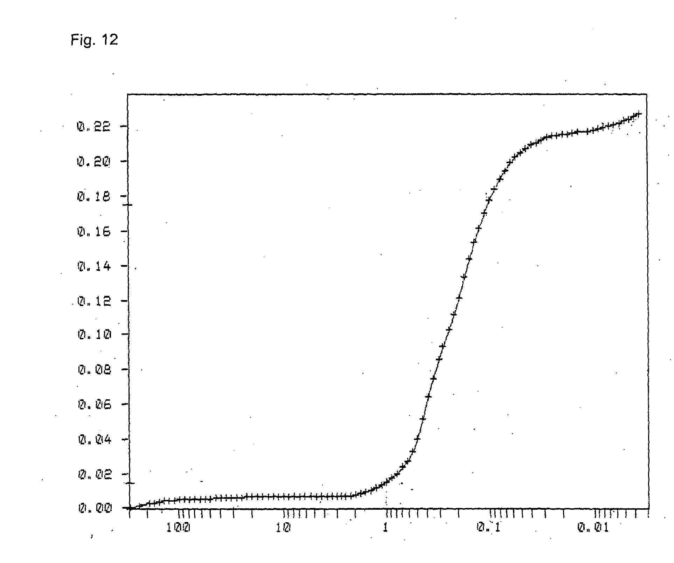

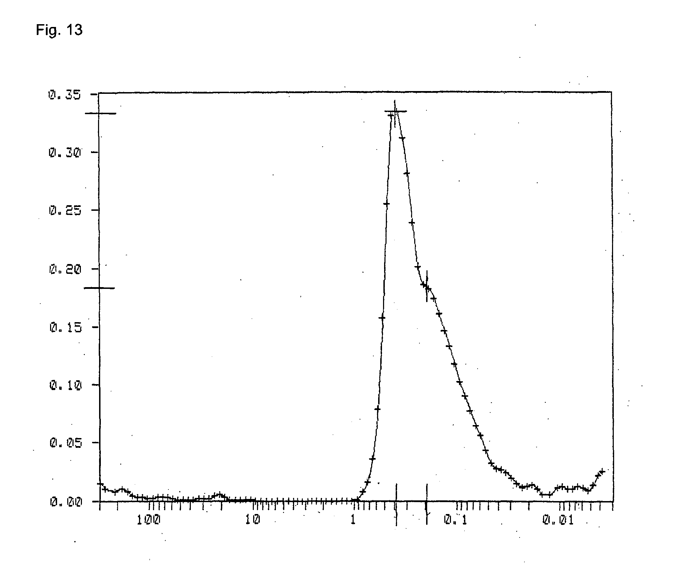

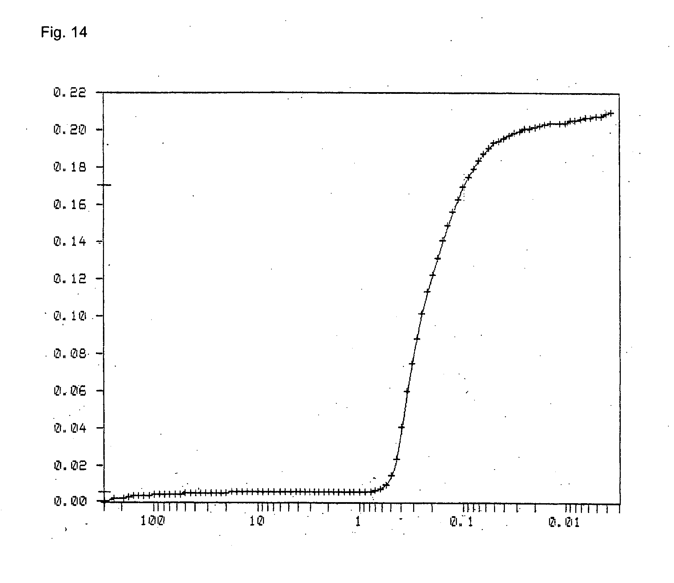

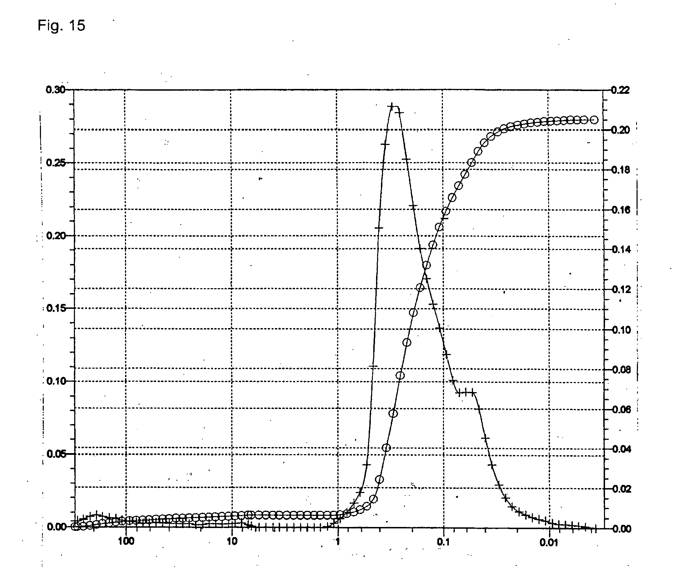

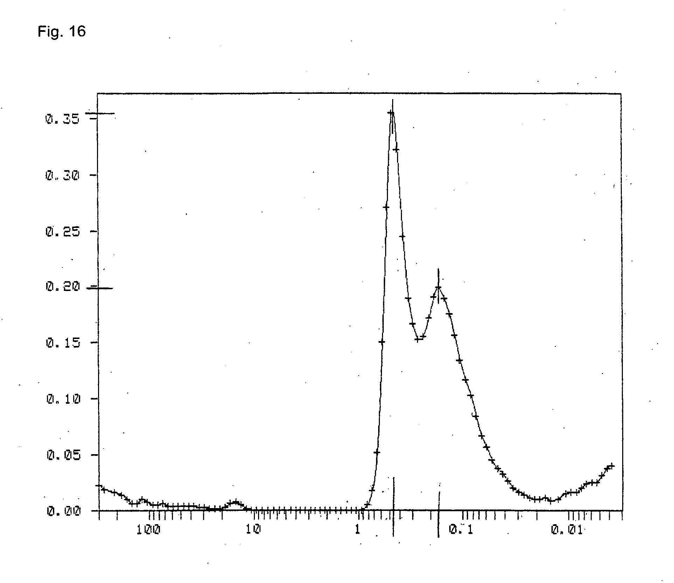

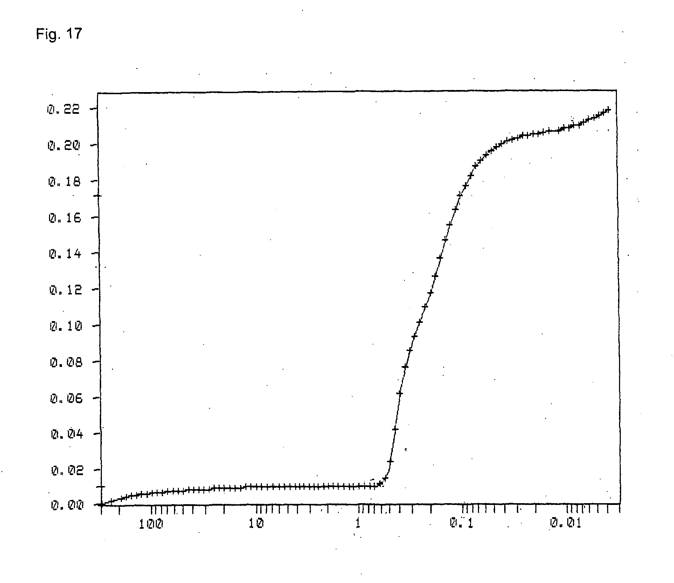

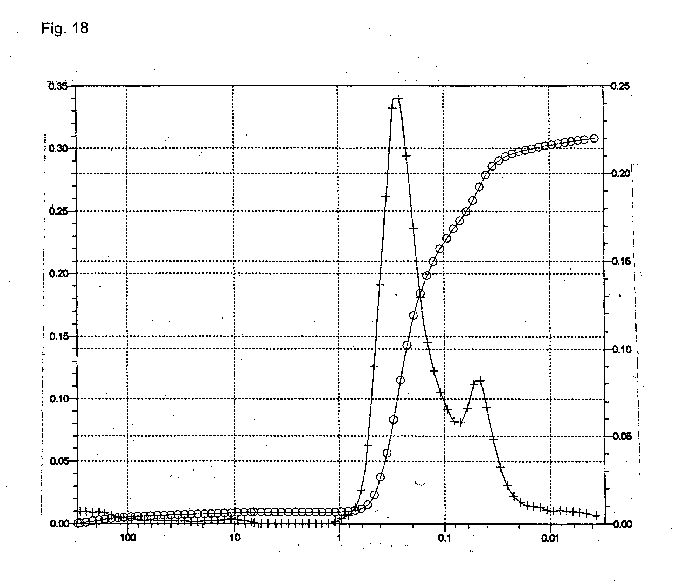

Entsprechende Figuren bilden die Figuren 11, 12 (VVK4), die Figuren 13, 14 (VVK6), die Figur 15 (VVK7), die Figuren 16, 17 (VVK8) und die Figur 18 (VVK9).

Anstatt die thermische Behandlung wie beschrieben durchzuführen, kann man sie auch wie in Beispiel 1 der DE-A 100 46 957 (die Schütthöhe in der Zersetzung (Kammern 1 bis 4) beläuft sich dabei jedoch vorteilhaft auf 44 mm bei einer Verweilzeit pro Kammer von 1,46 h und in der Calcination (Kammern 5 bis 8) beläuft sie sich vorteilhaft auf 130 mm bei einer Verweilzeit von 4,67 h) beschrieben mittels einer Bandcalziniervorrichtung durchführen; die Kammern besitzen eine Grundfläche (bei einer einheitlichen Kammerlänge von 1,40 m) von 1,29 m² (Zersetzung) und 1,40 m² (Calcination) und werden von unten durch das grobmaschige Band von 75 Nm³/h Zuluft durchströmt, die mittels rotierender Ventilatoren angesaugt werden. Innerhalb der Kammern ist die zeitliche und örtliche Abweichung der Temperatur vom Sollwert stets ≤ 2°C. Im übrigen wird wie in Beispiel 1 der DE-A 100 46 957 beschrieben verfahren. Die resultierenden ringförmigen Vollkatalysatoren können wie die ringförmigen Vollkatalysatoren VVK3 bis VVK4 für die nachfolgend beschriebene gasphasenkatalytische Partialoxidation von Propen zu Acrolein eingesetzt werden.

Als weitere Alternative kann die thermische Behandlung in einem Umluftofen (z.B. in einem solchen der Fa. Elino vom Typ Laborkammerofen KA-040/006-08 EW.OH bzw. einem solchen der Fa. Heraeus vom Typ K 750) so durchgeführt werden, dass man innerhalb von 6 h auf 270°C erwärmt und anschließend so lange die 270°C aufrechterhält, bis die Umluft frei ist von nitrosen Gasen. Nachfolgend wird innerhalb von 1,5 h auf eine Temperatur von 430°C bis 460°C (vorzugsweise auf 438°C) erwärmt und diese Temperatur 10 h aufrechterhalten. Die Luftspülung beträgt 800 Nl/h. 1000 g ringförmige Vollkatalysatorvorläuferformkörper sind in einen quaderförmigen Drahtkorb (10 cm hoch, 14 cm x 14 cm Grundfläche) in einer Schütthöhe von ca. 4 cm eingefüllt. Die Restgrundfläche des Tragekorbes wird in entsprechender Schütthöhe mit Steatitringen (wie immer in den Beispielen und Vergleichsbeispielen vom Typ C220 der Fa. Ceram Tec, DE) gleicher Geometrie belegt.

Diese thermischen Behandlungsbedingungen können auch auf die ringförmigen Vollkatalysatorvorläuferformkörper VVV1 und VVV2 angewendet werden. Alle resultierenden ringförmigen Vollkatalysatoren können in der unter C) beispielhaft beschriebenen gasphasenkatalytischen Partialoxidation eingesetzt werden.

### C) Testung der in I. A) und I. B) hergestellten ringförmigen Vollkatalysatoren für eine heterogen katalysierte Partialoxidation von Propen zu Acrolein

### 1. Versuchsanordnung

Ein Reaktionsrohr (V2A Stahl; 21 mm Außendurchmesser, 3 mm Wandstärke, 15 mm Innendurchmesser, Länge 100 cm) wurde von oben nach unten in Strömungsrichtung wie folgt beschickt:

| | |
|---|---|
| Abschnitt 1: | 30 cm Länge Steatitringe der Geometrie 5 mm x 3 mm x 2 mm (Außendurchmesser x Länge x Innendurchmesser) als Vorschüttung. |
| Abschnitt 2: | 70 cm Länge Katalysatorbeschickung mit den in A) und B) hergestellten ringförmigen Vollkatalysatoren. |

Die Temperierung des Reaktionsrohres erfolgte mittels eines mit Stickstoff geperiten Salzbades.

### 2. Versuchsdurchführung

Die beschriebene, jeweils frisch hergerichtete, Versuchsanordnung wurde jeweils kontinuierlich mit einem Beschickungsgasgemisch (Gemisch aus Luft, polymer grade Propylen und Stickstoff) der Zusammensetzung:
5 Vol.-% Propen,
10 Vol.-% Sauerstoff und
als Restmenge bis 100 Vol.-% N₂
beschickt, wobei die Belastung und die Thermostatisierung des Reaktionsrohres so erfolgte, dass der Propenumsatz U (mol-%) bei einmaligem Durchgang des Beschickungsgasgemisches durch das Reaktionsrohr kontinuierlich etwa 95 mol-% betrug.

Die nachfolgende Tabelle zeigt die in Abhängigkeit von der gewählten Katalysatorbeschickung und Propenbelastung (PL in Nl/l • h) derselben zur Umsatzerlangung erforderlichen Salzbadtemperaturen T_{S} (°C) sowie die dabei erzielten Acroleinselektivitäten S^{A} (mol-%). Die angegebenen Ergebnisse beziehen sich stets auf das Ende einer Betriebsdauer von 120 h. Die Selektivität S^{AA} der Acrylsäurenebenproduktbildung lag im Bereich von 4 bis 17 mol-%.

| ringförmiger Vollkatalysator | PL | T_{S} | S^{A} | S^{AA} |
|---|---|---|---|---|
| VVK1 | 50 | 306 | 89,5 | 4,7 |
| VVK2 | 50 | 306 | 89,5 | 4,6 |
| VVK1 | 75 | 310 | 90,5 | 4,9 |
| VVK2 | 75 | 311 | 90,5 | 4,9 |
| VVK1 | 100 | 315 | 90,8 | 5,2 |
| VVK2 | 100 | 318 | 91,1 | 5,1 |
| VVK3 | 50 | 320 | 88,6 | 7,1 |
| VVK4 | 50 | 325 | 86,1 | 8,8 |
| VVK5 | 50 | 322 | 86,6 | 8,9 |
| VVK6 | 50 | 338 | 84,9 | 10,2 |
| VVK7 | 50 | 320 | 90,2 | 5,1 |
| VVK8 | 50 | 343 | 85,0 | 10,3 |
| VVK9 | 50 | 322 | 90,0 | 5,4 |
| VVK10 | 50 | 333 | 93,1 | 5,4 |
| VVK11 | 50 | 333 | 87,9 | 7,6 |

Die vorstehenden Versuche können aber auch in entsprechender Weise (gleicher Zielumsatz) in einem Reaktionsrohr der nachfolgenden Art durchgeführt werden: V2A Stahl; 30 mm Außendurchmesser, 2 mm Wandstärke, 26 mm Innendurchmesser, 350 cm Länge, ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann.

In Strömungsrichtung wird dabei wie folgt beschickt:

| | |
|---|---|
| Abschnitt 1: | 80 cm Länge Steatitringe der Geometrie 7 mm x 7 mm x 4 mm (Außendurchmesser x Länge x Innendurchmesser) als Vorschüttung. |
| | |
| Abschnitt 2: | 270 cm Länge Katalysatorbeschickung mit den in A) und B) hergestellten ringförmigen Vollkatalysatoren. |

Die Temperierung des Reaktionsrohres erfolgt mittels eines im Gegenstrom gepumpten Salzbades.

PL wird durchgehend zu 100 gewählt. Die Zusammensetzung des Reaktionsgasausgangsgemisches ist 5,4 Vol.-% Propen, 10,5 Vol.-% Sauerstoff, 1,2 Vol.-% COₓ, 81,3 Vol.-% N₂ und 1,6 Vol.-% H₂O.

Diese Versuchsdurchführung kann in entsprechender Weise auch mit einer Katalysatorbeschickung durchgeführt werden, deren Abschnitt 2 wie folgt gestaltet ist (jeweils in Strömungsrichtung):
I. Zunächst auf 100 cm Länge ein homogenes Gemisch aus 65 Gew.-% VVK3 und 35 Gew.-% Steatitringen (5 mm x 3 mm x 2 mm);
   anschließend auf 170 cm Länge ein homogenes Gemisch aus 90 Gew.-% VVK3 und 10 Gew.-% Steatitringen (5 mm x 3 mm x 2 mm);
   oder
II. Zunächst auf 100 cm Länge VVK10; anschließend auf 170 cm Länge VVK3;
   oder
III. Zunächst auf 100 cm Länge ein homogenes Gemisch aus 70 Gew.-% VVK3 und 30 Gew.-% Steatitringen (5 mm x 3 mm x 2 mm); anschließend auf 170 cm Länge VVK3.
Tₛ wird in allen Fällen so gewählt, dass U-Propen = 95 mol-%.

### II. Beispiele für eine heterogen katalysierte Partialoxidation von Propen zu Acrolein

Die ringförmigen Vollkatalysatoren VVK1 bis VVK11 wurden wie in I. beschrieben nochmals hergestellt (d.h., mit identischer Aktivmassenzusammensetzung), jedoch mit dem Unterschied, dass das bei den Herstellungen in I. als Hilfsmittel mitverwendete TIMREX T44 der Firma Timcal AG in allen Fällen durch eine entsprechende Gewichtsmenge des in dieser Schrift bereits beschriebenen Boron Nitride Grade A 01, Number PD-5006, Issue 0-07.99, HS Number: 28500030 der Fa. H.C. Starck ersetzt wurde.

Es wurden so ringförmige Vollkatalysatoren BVK1 bis BVK11 erhalten (der erste Buchstabe B steht jeweils für Beispiel), deren physikalische Eigenschaften (z.B. O, V, d^{max}, V^{0,1}₁ u. R) von jenen der Vergleichsvollkatalysatoren VVK1 bis VVK11 innerhalb der Reproduzierbarkeitsgrenzen nicht unterscheidbar waren.

Desgleichen ergab sich bei der Testung der ringförmigen Vollkatalysatoren BVK1 bis BVK11 als Katalysatoren für eine heterogen katalysierte Partialoxidation von Propen zu Acrolein unter den in I. C) beschriebenen Testbedingungen für die Werte von Tₛ, S^{A} und S^{AA}. Die für die Vergleichsvollkatalysatoren VVK1 bis VVK11 in I. beschriebenen Verfahrensvarianten im Rahmen ihrer Herstellung und/oder Testung gelten so auch für die Vollkatalysatoren BVK1 bis BVK11.

### III. Beispiele für eine heterogen katalysierte Partialoxidation von Methacrolein zu Methacrylsäure

### A) Herstellung von ringförmigen Vollkatalysatoren mit der nachfolgenden Stöchiometrie Mo₁₂P_{1,5}V_{0,6}Cs_{1,0}Cu_{0,5}Sb₁S_{0,04}Oₓ der Aktivmasse.

In 619 I auf 45 °C temperiertes Wasser in einem wassertemperierten Doppelmantelbehälter wurden unter Rühren (70 Umdrehungen pro Minute (UPM)) 537,5 kg Ammoniumheptamolybdattetrahydrat ((NH₄)₆Mo₇O₂₄ · 4 H₂O (81 Gew.-% MoO₃, 8 Gew.-% NH₃, ≤ 50 Gew.-ppm Na und ≤ 100 Gew.-ppm K) eindosiert. Die Temperatur der Lösung sank hierbei auf 37 °C ab. Um ein sicheres Auflösen des Ammoniumheptamolybdats zu gewährleisten, wurde nach Ende der Zudosierung noch 15 Minuten nachgerührt, wobei die Temperatur von 37 °C beibehalten wurde. Unter weiterem Rühren wurden bei derselben Temperatur innerhalb von 3 Minuten 17,82 kg Ammoniummetavanadat (NH₄VO₃, 77 Gew.-% V₂O₅, 14,5 Gew.-% NH₃, ≤ 150 Gew.-ppm Na und ≤ 500 Gew.-ppm K) zudosiert. Es wurde 2 Minuten nachgerührt. Dann wurde innerhalb einer Minute eine in einem separaten Lösebehälter hergestellte, farblose, klare, 60°C warme Lösung von 49,6 kg Cäsiumnitrat (CsNO₃ mit 72 Gew.-% Cs₂O und ≤ 50 Gew.-ppm Na, ≤ 100 Gew.-ppm K, ≤ 10 Gew.-ppm Al sowie ≤ 20 Gew.-ppm Fe) in 106 I Wasser eingerührt. Hierbei stieg die Temperatur der resultierenden Suspension auf 39 °C. Nach einminütigem Nachrühren wurden innerhalb einer weiteren Minute unter fortgesetztem Rühren 31,66 I 75 gew.-%ige Phosphorsäure (Dichte bei 25 °C und 1 atm: 1,57 g/ml, Viskosität bei 25°C und 1 atm: 0,147 cm²/S) zudosiert. Aufgrund der exothermen Reaktion stieg die Temperatur hierbei auf 42°C. Erneut wurde 1 Minute nachgerührt. Dann wurden innerhalb einer Minute 1,34 kg Ammoniumsulfat ((NH₄)₂SO₄ (> 99 Gew.-%)) eingerührt und 1 weitere Minute nachgerührt. Unter fortgesetztem Rühren bei identischer Temperatur wurden innerhalb von 3 Minuten 37,04 kg Antimontrioxid (Sb₂O₃, mittlerer Partikeldurchmesser D₅₀ = ca. 2µm, Kristallstruktur laut XRD: > 75 % Senarmontit, < 25 % Valentinit, Reinheit: > 99,3 Gew.-%, ≤ 0,3 Gew.-% As₂O₃, ≤ 0,3 Gew.-% PbO und ≤ 300 Gew.-ppm FeO) zugegeben (käuflich erhältlich als Triox White, Code No. 639000 der Fa. Antraco, D-10407 Berlin). Nun wurde die Rührerdrehzahl von 70 auf 50 UPM zurückgenommen. Anschließend wurde die gerührte Suspension mittels Dampf im Doppelmantel innerhalb von 30 Minuten linear auf 95°C aufgeheizt. Bei dieser Temperatur und 50 UPM wurden innerhalb von 4 Minuten 51,64 kg Kupfernitratlösung (wässrige Cu(NO₃)₂-Lösung mit 15,6 Gew.-% Cu) zugegeben. Nach vierminütigem Nachrühren bei 95 °C wurde die Rührgeschwindigkeit weiter von 50 auf 35 UPM zurückgenommen. Anschließend wurde die gesamte Suspension innerhalb von 4 Minuten in einen mit Stickstoff überlagerten, auf 85 °C temperierten und mit 35 UPM gerührten Sprühturmvorlagenbehälter abgelassen und es wurde mit 20 I Wasser (25 °C) nachgespült. Aus diesem heraus wurde die Suspension in einem Drehscheibensprühturm im Gegenstrom mit einer Eintrittstemperatur von 285°C und einer Austrittstemperatur von.110°C innerhalb von 3,5 h sprühgetrocknet, wobei das resultierende Sprühpulver einen Glühverlust (1 h bei 500 °C in Luft) von ca. 16 Gew.-% aufwies.

Das Sprühpulver wurde mit 1,5 Gew.-% Bornitrid (Boron Nitrade Grade A01, Number PD-5006, Issue 0-07.99 der Fa. H.C. Starck) homogen vermischt und kompaktiert (Kompaktor der Fa. Hosokawa Bepex GmbH, D-74211 Leingarten, Typ K200/100 mit konkaven, geriffelten Glattwalzen, Spaltweite: 2,8 mm, Siebweite: 1,25 mm, Siebweite Unterkorn: 400 µm, Schneckendrehzahl: 65 bis 70 UPM). Für die Tablettierung wurden dem Kompaktat weitere 1 Gew.-% desselben Bornitrids zugemischt. Anschließend wurde das Kompaktat in einem Kilian Rundläufer (Tablettiermaschine vom Typ R x 73 der Fa. Kilian, D-50735 Köln) unter Stickstoffatmosphäre zu ringförmigen Vollringtabletten der Geometrie 7 mm x 7 mm x 3 mm (Außendurchmesser x Länge x Innendurchmesser) mit einer Seitendruckfestigkeit von 35 ± 2 N tablettiert.

8 kg der Rohtabletten wurden in einem Drahtbehälter der Grundfläche 33,0 cm x 49,5 cm gleichmäßig verteilt, wobei sich eine Schütthöhe von 4 cm ergab. Der Drahtbehälter wurde in einem Kammerofen (Fa. Elino Industrie-Ofenbau, Carl Hanf GmbH & Co, D-52355 Düren, Typ KA-040/006-08 EW.OH, Abmessungen: Länge = 57 cm, Breite = 57 cm, Höhe = 80 cm) so angeordnet, dass die Schüttung der Tabletten gleichmäßig durchströmbar war. Es wurden 2 Nm³/h Frischluft zugeführt und die Luftumwälzung im Ofen so eingestellt, dass die Schüttung mit einer Geschwindigkeit von 0,9 m/s (bestimmt mittels Aerometer, Fa. Testo, Typ 445) durchströmt wurde. Der Ofen wurde nun mit der folgenden Temperaturrampe auf 380 °C aufgeheizt: innerhalb von 40 min auf 180 °C aufheizen, 30 min halten, innerhalb von 10 min auf 220 °C aufheizen, 30 min halten, innerhalb von 13 min auf 270 °C aufheizen, 30 min halten, innerhalb von 25 min auf 340 °C und dann innerhalb von 40 min auf 380 °C aufheizen. Diese Temperatur wurde dann 390 min gehalten. Währenddessen wurde der NH₃-Gehalt in der abgesaugten Atmosphäre der thermischen Behandlung kontinuierlich durch FTIR-Spektroskopie überwacht (Spektrometer der Fa. Nicolet, Typ "Impact", IR-Edelstahlzelle mit CaF₂-Fenster, 10 cm Schichtdicke, Temperierung auf 120 °C, Bestimmung der Konzentration anhand der Intensität der Bande bei 3.333 cm⁻¹). Der NH₃-Gehalt blieb während der gesamten thermischen Behandlung ≤ 2,4 Vol%. Dieser Maximalwert wurde bei 220 °C erreicht. Die so erhaltenen ringförmigen Katalysatorformkörper BVK12 wiesen eine Seitendruckfestigkeit von 15 ± 2 N, einen Ammonium-Gehalt (bestimmt durch Titration nach Kjeldahl) von 0,3 Gew.-% NH₄⁺ und einen MoO₃-Gehalt von 2 XRD-Intensitäts-% auf. Letzterer berechnet sich als Verhältnis der Intensität des (021)-MoO₃-Reflexes bei 2⊝ = 27,3° zur Intensität des (222)-Reflexes der Heteropolyverbindung bei 2⊝ = 26,5° im Röntgenpulverdiffraktogramm (mit Cu-Kα-Strahlung).

(Alternativ zur beschriebenen Calcinierung im Kammerofen kann die Calcinierung auch hier, wie im Beispiel I.A beschrieben, im Bandcalzinierer erfolgen.)

### B) Testung der ringförmigen Vollkatalysatoren aus II. A für eine heterogen katalysierte Partialoxidation von Methacrolein zu Methacrylsäure

2 kg der so hergestellten ringförmigen Katalysatorformkörper BVK12 wurden mit einer Vor- und einer Nachschüttung von jeweils 50 g Steatitringen (Steatit C220 der Fa. CeramTec) der Geometrie 7 mm x 7 mm x 4 mm Außendurchmesser x Länge x Innendurchmesser in ein Modellrohr aus Edelstahl (Außendurchmesser = 30 mm, Innendurchmesser = 26 mm, Länge = 4,15 m) gefüllt (Füllhöhe: 397 cm). Dieses befand sich in einem mit Stickstoff geperiten, auf 287°C temperierten Salzbad. Die katalytische Testung erfolgte unter Kreisgasfahrweise: Das Reaktoraustrittsgas wurde hierbei auf eine Venturidüse gefahren, dort mit 75 °C warmem Wasser abgeschreckt und dann in den Sumpf der auf 75°C temperierten Destillationskolonne A geleitet. Ca. 55 kg/Tag einer Mischung aus Reaktionsprodukt und Wasser (typischerweise ca. 9,5 Gew.-% Methacrylsäure, ca. 0,8 Gew.-% Essigsäure und ca. 0,1 Gew.-% Acrylsäure in Wasser) wurden hier entnommen. Der abgereicherte Gasstrom gelangte in die Kolonne A. In deren Mitte wurde ein Teilstrom entnommen und unten in eine auf 7°C temperierte Kolonne B geleitet. Mit einer am Kopf der Kolonne B eingespeisten Lösung von 6 Gew.-% Hydrochinon in Wasser (2 kg/h) wurde das Abgas in dieser Kolonne von den verbliebenen organischen Komponenten befreit und entwich am Kollonnenkopf. Der Inhalt des Sumpfs der Kolonne B (im wesentlichen ca. 1,4 Gew.-% Methacrolein in Wasser) wurde an den Kopf der Kolonne A gepumpt; dort wurden zusätzlich 220 g/h Methacrolein eingespeist. Am Kopf der Kolonne A wurden bei einer Kopftemperatur von 66°C 1700 Nl/h Kreisgas entnommen, mit 450 Nl/h Frischluft gemischt und als E-duktgas das ca. 5 Vol.-% Methacrolein, ca. 12 Vol.-% O₂, ca. 21 Vol.-% Wasserdampf, ca. 2,5 Vol.-% CO, ca. 3 Vol.-% CO₂ und weiteres Inertgas (im wesentlichen Stickstoff) enthielt, in den Reaktor geleitet. Damit ergab sich eine massenbezogene Raumgeschwindigkeit (WHSV) von 0,17 h⁻¹.

Während der 5-tägigen Testung wurde der Methacrolein-Umsatz im einfachen Durchgang bei 65 mol-% gehalten; hierzu wurde die Salzbadtemperatur schrittweise auf 291 °C erhöht. Am 5. Tag ergab sich eine Selektivität zu Methacrylsäure von 85,0 mol-%. Als Nebenprodukte bildeten sich (mit Angabe der Selektivitäten) 4,8 mol-% CO₂, 4,8 mol-% Essigsäure, 4,1 mol-% CO, 0,7 mol-% Acrylsäure und 0,6 mol-% Maleinsäure.

### IV. Vergleichsbeispiele für eine heterogen katalysierte Partialoxidation von Methacrolein zu Methacrylsäure

Der ringförmige Vollkatalysator BVK12 aus III. wurde wie in III. beschrieben nochmals hergestellt (d.h., mit identischer Aktivmassenzusammensetzung), jedoch mit dem Unterschied, dass das bei der Herstellung in III. als Hilfsmittel mitverwendete Bornitrid durch entsprechende Gewichtsmengen des Graphit TIMREX T44 der Firma Timcal AG ersetzt wurde.

Die so erhaltenen ringförmigen Vergleichsvollkatalysatoren VVK12 wiesen einen Ammonium-Gehalt von 0,3 Gew.-% NH₄⁺ und einen MoO₃-Gehalt von 2 XRD-Intensitäts-% auf.

Sie wurden anschließend wie in III. B beschrieben getestet. Am 5. Tag der katalytischen Testung unter den in III. B beschriebenen Bedingungen wurde eine Salzbadtemperatur von 292°C benötigt. Es ergab sich eine Selektivität zu Methacrylsäure von 84,6 mol-%, als Nebenprodukte bildeten sich (mit Angabe der Selektivitäten) 5,0 mol-% CO₂, 4,8 mol-% Essigsäure, 4,3 mol-% CO, 0,7 mol-% Acrylsäure und 0,6 mol-% Maleinsäure.

## Patentansprüche

1. Verfahren zur Herstellung von Katalysatorformkörpern, deren Aktivmasse ein Multielementoxid ist, bei dem man ein feinteiliges Vorläufergemisch, das ein feinteiliges Formungshilfsmittel zugesetzt enthält, zur gewünschten Geometrie formt und die dabei resultierenden Katalysatorvorläuferformkörper bei erhöhter Temperatur unter Erhalt der Katalysatorformkörper, deren Aktivmasse ein Multielementoxid ist, thermisch behandelt, **dadurch gekennzeichnet, dass** das feinteilige Vorläufergemisch als Formungshilfsmittel Bornitrid zugesetzt enthält.

2. Verfahren nach Anspruch 1, **dadurch gekennzeichnet, dass** das zugesetzte Bornitrid ≤ 5 Gew.-% an B₂O₃ enthält.

3. Verfahren nach Anspruch 1, **dadurch gekennzeichnet, dass** das zugesetzte Bornitrid ≤ 3 Gew.-% an B₂O₃ enthält.

4. Verfahren nach Anspruch 1**, dadurch gekennzeichnet, dass** das zugesetzte Bornitrid ≤ 1 Gew.-% B₂O₃ enthält.

5. Verfahren nach einem der Ansprüche 1 bis 4, **dadurch gekennzeichnet, dass** das zugesetzte Bornitrid ≥ 0,05 Gew.-% an B₂O₃ enthält.

6. Verfahren nach einem der Ansprüche 1 bis 5, **dadurch gekennzeichnet, dass** die Partikeldurchmesser des zugesetzten Bornitrid im Bereich 1 µm bis 50 µm liegen.

7. Verfahren nach einem der Ansprüche 1 bis 5, **dadurch gekennzeichnet, dass** die Partikeldurchmesser des zugesetzten Bornitrid im Bereich 1 µm bis 10 µm liegen.

8. Verfahren nach einem der Ansprüche 1 bis 7, **dadurch gekennzeichnet, dass** das feinteilige Vorläufergemisch, bezogen auf sein Gesamtgewicht, 0,1 bis 20 Gew.-% Bornitrid zugesetzt enthält.

9. Verfahren nach einem der Ansprüche 1 bis 7, **dadurch gekennzeichnet, dass** das feinteilige Vorläufergemisch, bezogen auf sein Gesamtgewicht, 0,3 bis 8 Gew.-% Bornitrid zugesetzt enthält.

10. Verfahren nach einem der Ansprüche 1 bis 9, **dadurch gekennzeichnet, dass** das zugesetzte Bornitrid zu wenigstens 50 Gew.-% in hexagonaler Phase vorliegt.

11. Verfahren nach einem der Ansprüche 1 bis 9, **dadurch gekennzeichnet, dass** das zugesetzte Bornitrid zu wenigstens 75 Gew.-% in hexagonaler Phase vorliegt.

12. Verfahren nach einem der Ansprüche 1 bis 11, **dadurch gekennzeichnet, dass** das zugesetzte Bornitrid wie folgt beschaffen ist:
| | |
|---|---|
| Partikeldurchmesser: | 1 bis 10 µm, |
| spezifische Oberfläche: | 5 bis 20 m²/g, |
| Schüttdichte: | 0,2 bis 0,6 g/cm³, und |
| Klopfdichte: | 0,3 bis 0,7 g/cm³. |

13. Verfahren nach einem der Ansprüche 1 bis 11, **dadurch gekennzeichnet, dass** das zugesetzte Bornitrid wie folgt beschaffen ist:
| | |
|---|---|
| Partikeldurchmesser: | 1 bis 5 µm, |
| spezifische Oberfläche: | 5 bis 15 m²/g, |
| Schüttdichte: | 0,2 bis 0,6 g/cm³, und |
| Klopfdichte: | 0,3 bis 0,7 g/cm³. |

14. Verfahren nach einem der Ansprüche 1 bis 13, **dadurch gekennzeichnet, dass** die thermische Behandlung der Katalysatorvorläuferformkörper bei Temperaturen von 150°C bis 650°C erfolgt.

15. Verfahren nach einem der Ansprüche 1 bis 14, **dadurch gekennzeichnet, dass** die thermische Behandlung der Katalysatorvorläuferformkörper in oxidierender Atmosphäre erfolgt.

16. Verfahren nach einem der Ansprüche 1 bis 14, **dadurch gekennzeichnet, dass** die thermische Behandlung der Katalysatorvorläuferformkörper im Luftstrom erfolgt.

17. Verfahren nach einem der Ansprüche 1 bis 16, **dadurch gekennzeichnet, dass** die Partikeldurchmesser des feinteilige Vorläufergemischs, das zugesetzte Formungshilfsmittel ausgenommen, im Bereich 1 bis 2000 µm liegen.

18. Verfahren nach einem der Ansprüche 1 bis 17, **dadurch gekennzeichnet, dass** die Formung zur gewünschten Geometrie durch Tablettieren erfolgt.

19. Verfahren nach einem der Ansprüche bis 1 bis 18, **dadurch gekennzeichnet, dass** die Formung unter Anwendung eines Formgebungsdrucks von 50 kg/cm² bis 5000 kg/cm² erfolgt.

20. Verfahren nach einem der Ansprüche 1 bis 19, **dadurch gekennzeichnet, dass** der Katalysatorformkörper eine Kugel mit einem Durchmesser von 2 bis 10 mm ist.

21. Verfahren nach einem der Ansprüche 1 bis 19, **dadurch gekennzeichnet, dass** der Katalysatorformkörper ein Vollzylinder ist, dessen Außendurchmesser und Länge 2 bis 10 mm betragen.

22. Verfahren nach einem der Ansprüche 1 bis 19, **dadurch gekennzeichnet, dass** der Katalysatorformkörper ein Ring ist, dessen Außendurchmesser und Länge 2 bis 10 mm betragen und dessen Wandstärke 1 bis 3 mm beträgt.

23. Verfahren nach einem der Ansprüche 1 bis 22, **dadurch gekennzeichnet, dass** die Aktivmasse ein Multimetalloxid ist.

24. Verfahren nach einem der Ansprüche 1 bis 22, **dadurch gekennzeichnet, dass** die Aktivmasse eine Multielementoxidmasse ist, die
a) die Elemente Mo, Fe und Bi, oder
b) die Elemente Mo und V, oder
c) die Elemente Mo, V und P, oder
d) die Elemente V und P
umfasst.

25. Katalysatorformkörper, erhältlich nach einem Verfahren gemäß einem der Ansprüche 1 bis 24.

26. Katalysatorformkörper, dessen Aktivmasse ein Multielementoxid ist, das 0,1 bis 20 Gew.-% an Bornitrid enthält.

27. Verfahren einer heterogen katalysierten Gasphasenreaktion, **dadurch gekennzeichnet, dass** der Katalysator wenigstens einen Katalysatorformkörper gemäß Anspruch 25 oder 26 umfasst.

28. Verfahren nach Anspruch 27, **dadurch gekennzeichnet, dass** die heterogen katalysierte Gasphasenreaktion eine heterogen katalysierte Partialoxidation einer organischen Verbindung ist.

29. Verfahren nach Anspruch 28, **dadurch gekennzeichnet, dass** die heterogen katalysierte Partialoxidation die partielle Oxidation von Propen zu Acrolein und/oder Acrylsäure ist.

30. Verfahren nach Anspruch 28, **dadurch gekennzeichnet, dass** die heterogen katalysierte Partialoxidation die partielle Oxidation von Propan zu Acrylsäure ist.

31. Verfahren nach Anspruch 28, **dadurch gekennzeichnet, dass** die heterogen katalysierte Partialoxidation die partielle Oxidation von Methacrolein zu Methacrylsäure ist.

32. Verfahren nach Anspruch 28, **dadurch gekennzeichnet, dass** die heterogen katalysierte Partialoxidation die partielle Oxidation eines Kohlenwasserstoffs mit mindestens 4 Kohlenstoffatomen zu Maleinsäureanhydrid ist.

33. Verfahren nach Anspruch 28, **dadurch gekennzeichnet, dass** die heterogen katalysierte Partialoxidation die partielle Oxidation von iso-Buten zu Methacrolein ist.

## Claims

1. A process for preparing shaped catalyst bodies whose active composition is a multielement oxide, in which a finely divided precursor mixture which comprises an added finely divided shaping assistant is shaped to the desired geometry and the resulting shaped catalyst precursor bodies are treated thermally at elevated temperature to obtain the shaped catalyst bodies whose active composition is a multielement oxide, wherein the finely divided precursor mixture comprises added boron nitride as the shaping assistant.

2. The process according to claim 1, wherein the added boron nitride comprises ≤ 5% by weight of B₂O₃.

3. The process according to claim 1, wherein the added boron nitride comprises ≤ 3% by weight of B₂O₃.

4. The process according to claim 1, wherein the added boron nitride comprises ≤ 1% by weight of B₂O₃.

5. The process according to any of claims 1 to 4, wherein the added boron nitride comprises ≥ 0.05% by weight of B₂O₃.

6. The process according to any of claims 1 to 5, wherein the particle diameter of the added boron nitride is within the range from 1 µm to 50 µm.

7. The process according to any of claims 1 to 5, wherein the particle diameter of the added boron nitride is within the range from 1 µm to 10 µm.

8. The process according to any of claims 1 to 7, wherein the finely divided precursor mixture, based on its total weight, comprises from 0.1 to 20% by weight of added boron nitride.

9. The process according to any of claims 1 to 7, wherein the finely divided precursor mixture, based on its total weight, comprises from 0.3 to 8% by weight of added boron nitride.

10. The process according to any of claims 1 to 9, wherein the added boron nitride is present to an extent of at least 50% by weight in hexagonal phase.

11. The process according to any of claims 1 to 9, wherein the added boron nitride is present to an extent of at least 75% by weight in hexagonal phase.

12. The process according to any of claims 1 to 11, wherein the added boron nitride has the following properties:
| | |
|---|---|
| particle diameter: | from 1 to 10 µm, |
| specific surface area: | from 5 to 20 m²/g, |
| bulk density: | from 0.2 to 0.6 g/cm³, and |
| tap density: | from 0.3 to 0.7 g/cm³. |

13. The process according to any of claims 1 to 11, wherein the added boron nitride has the following properties:
| | |
|---|---|
| particle diameter: | from 1 to 5 µm, |
| specific surface area: | from 5 to 15 m²/g, |
| bulk density: | from 0.2 to 0.6 g/cm³, and |
| tap density: | from 0.3 to 0.7 g/cm³. |

14. The process according to any of claims 1 to 13, wherein the shaped catalyst precursor bodies are treated thermally at temperatures of from 150°C to 650°C.

15. The process according to any of claims 1 to 14, wherein the shaped catalyst precursor bodies are treated thermally in oxidizing atmosphere.

16. The process according to any of claims 1 to 14, wherein the shaped catalyst precursor bodies are treated thermally in an air stream.

17. The process according to any of claims 1 to 16, wherein the particle diameters of the finely divided precursor mixture, excluding the added shaping assistant, are within the range from 1 to 2000 µm.

18. The process according to any of claims 1 to 17, wherein the shaping to the desired geometry is effected by tableting.

19. The process according to any of claims 1 to 18, wherein the shaping is effected with application of a shaping pressure of from 50 kg/cm² to 5000 kg/cm².

20. The process according to any of claims 1 to 19, wherein the shaped catalyst body is a sphere having a diameter of from 2 to 10 mm.

21. The process according to any of claims 1 to 19, wherein the shaped catalyst body is a solid cylinder whose external diameter and length are from 2 to 10 mm.

22. The process according to any of claims 1 to 19, wherein the shaped catalyst body is a ring whose external diameter and length are from 2 to 10 mm and whose wall thickness is from 1 to 3 mm.

23. The process according to any of claims 1 to 22, wherein the active composition is a multimetal oxide.

24. The process according to any of claims 1 to 22, wherein the active composition is a multielement oxide composition which comprises
a) the elements Mo, Fe and Bi or
b) the elements Mo and V or
c) the elements Mo, V and P or
d) the elements V and P.

25. A shaped catalyst body obtainable by a process according to any of claims 1 to 24.

26. A shaped catalyst body whose active composition is a multielement oxide which comprises from 0.1 to 20% by weight of boron nitride.

27. A process for a heterogeneously catalyzed gas phase reaction, wherein the catalyst comprises at least one shaped catalyst body according to claim 25 or 26.

28. The process according to claim 27, wherein the heterogeneously catalyzed gas phase reaction is a heterogeneously catalyzed partial oxidation of an organic compound.

29. The process according to claim 28, wherein the heterogeneously catalyzed partial oxidation is the partial oxidation of propene to acrolein and/or acrylic acid.

30. The process according to claim 28, wherein the heterogeneously catalyzed partial oxidation is the partial oxidation of propane to acrylic acid.

31. The process according to claim 28, wherein the heterogeneously catalyzed partial oxidation is the partial oxidation of methacrolein to methacrylic acid.

32. The process according to claim 28, wherein the heterogeneously catalyzed partial oxidation is the partial oxidation of a hydrocarbon having at least 4 carbon atoms to maleic anhydride.

33. The process according to claim 28, wherein the heterogeneously catalyzed partial oxidation is the partial oxidation of isobutene to methacrolein.

## Revendications

1. Procédé pour la production de corps façonnés de catalyseur, dont la masse active est un oxyde de plusieurs éléments, dans lequel on façonne un mélange de précurseurs finement divisé, auquel on a ajouté un adjuvant de façonnage finement divisé, en une géométrie souhaitée et on traite thermiquement les corps façonnés de précurseur de catalyseur obtenu à température élevée avec obtention des corps façonnés de catalyseur, dont la masse active est un oxyde de plusieurs éléments, **caractérisé que** le mélange de précurseurs finement divisé contient du nitrure de bore ajouté comme adjuvant de façonnage finement divisé.

2. Procédé selon la revendication 1, **caractérisé en ce que** le nitrure de bore ajouté contient ≤ 5% en poids de B₂O₃.

3. Procédé selon la revendication 1, **caractérisé en ce que** le nitrure de bore ajouté contient ≤ 3% en poids de B₂O₃.

4. Procédé selon la revendication 1, **caractérisé en ce que** le nitrure de bore ajouté contient ≤ 1% en poids de B₂O₃.

5. Procédé selon l'une quelconque des revendications 1 à 4, **caractérisé en ce que** le nitrure de bore ajouté contient ≥ 0,05% en poids de B₂O₃.

6. Procédé selon l'une quelconque des revendications 1 à 5, **caractérisé en ce que** le diamètre des particules du nitrure de bore ajouté se situe dans la plage de 1 µm à 50 µm.

7. Procédé selon l'une quelconque des revendications 1 à 5, **caractérisé en ce que** le diamètre des particules du nitrure de bore ajouté se situe dans la plage de 1 µm à 10 µm.

8. Procédé selon l'une quelconque des revendications 1 à 7, **caractérisé en ce que** le mélange de précurseurs finement divisé, par rapport à son poids total, contient 0,1 à 20% en poids de nitrure de bore ajouté.

9. Procédé selon l'une quelconque des revendications 1 à 7, **caractérisé en ce que** le mélange de précurseurs finement divisé, par rapport à son poids total, contient 0,3 à 8% en poids de nitrure de bore ajouté.

10. Procédé selon l'une quelconque des revendications 1 à 9, **caractérisé en ce que** le nitrure de bore ajouté se trouve à raison d'au moins 50% en poids sous forme de phase hexagonale.

11. Procédé selon l'une quelconque des revendications 1 à 9, **caractérisé en ce que** le nitrure de bore ajouté se trouve à raison d'au moins 75% en poids sous forme de phase hexagonale.

12. Procédé selon l'une quelconque des revendications 1 à 11, **caractérisé en ce que** le nitrure de bore ajouté présente les caractéristiques suivantes :
| | |
|---|---|
| Diamètre de particules : | 1 à 10 µm, |
| Surface spécifique : | 5 à 20 m²/g, |
| Densité apparente : | 0,2 à 0,6 g/cm³, et |
| Densité tassée : | 0,3 à 0,7 g/cm³. |

13. Procédé selon l'une quelconque des revendications 1 à 11, **caractérisé en ce que** le nitrure de bore ajouté présente les caractéristiques suivantes :
| | |
|---|---|
| Diamètre de particules : | 1 à 5 µm, |
| Surface spécifique : | 5 à 15 m²/g, |
| Densité apparente : | 0,2 à 0,6 g/cm³, et |
| Densité tassée : | 0,3 à 0,7 g/cm³. |

14. Procédé selon l'une quelconque des revendications 1 à 13, **caractérisé en ce que** le traitement thermique des corps façonnés de précurseur de catalyseur est réalisé à des températures de 150°C à 650°C.

15. Procédé selon l'une quelconque des revendications 1 à 14, **caractérisé en ce que** le traitement thermique des corps façonnés de précurseur de catalyseur est réalisé dans une atmosphère oxydante.

16. Procédé selon l'une quelconque des revendications 1 à 14, **caractérisé en ce que** le traitement thermique des corps façonnés de précurseur de catalyseur est réalisé dans un flux d'air.

17. Procédé selon l'une quelconque des revendications 1 à 16, **caractérisé en ce que** les diamètres des particules du mélange de précurseurs finement divisé, à l'exception de l'adjuvant de façonnage, se situent dans la plage de 1 à 2000 µm.

18. Procédé selon l'une quelconque des revendications 1 à 17, **caractérisé en ce que** le façonnage en géométrie souhaitée est réalisé par compression en comprimés.

19. Procédé selon l'une quelconque des revendications 1 à 18, **caractérisé en ce que** le façonnage est réalisé avec utilisation d'une pression de façonnage de 50 kg/cm² à 5000 kg/cm².

20. Procédé selon l'une quelconque des revendications 1 à 19, **caractérisé en ce que** le corps façonné de catalyseur est une bille présentent un diamètre de 2 à 10 mm.

21. Procédé selon l'une quelconque des revendications 1 à 19, **caractérisé en ce que** le corps façonné de catalyseur est un cylindre plein dont le diamètre extérieur et la longueur sont de 2 à 10 mm.

22. Procédé selon l'une quelconque des revendications 1 à 19, **caractérisé en ce que** le corps façonné de catalyseur est un anneau dont le diamètre extérieur et la longueur sont de 2 à 10 mm et dont l'épaisseur de paroi est de 1 à 3 mm.

23. Procédé selon l'une quelconque des revendications 1 à 22, **caractérisé en ce que** la masse active est un oxyde de plusieurs métaux.

24. Procédé selon l'une quelconque des revendications 1 à 22, **caractérisé en ce que** la masse active est une masse d'oxyde de plusieurs métaux qui comprend
a) les éléments Mo, Fe et Bi, ou
b) les éléments Mo et V, ou
c) les éléments Mo, V et P, ou
d) les éléments V et P.

25. Corps façonné de catalyseur, pouvant être obtenu selon un procédé selon l'une quelconque des revendications 1 à 24.

26. Corps façonné de catalyseur dont la masse active est un oxyde de plusieurs éléments qui contient 0,1 à 20% en poids de nitrure de bore.

27. Procédé pour réaliser une réaction en phase gazeuse catalysée par voie hétérogène, **caractérisé en ce que** le catalyseur comprend au moins un corps façonné de catalyseur selon la revendication 25 ou 26.

28. Procédé selon la revendication 27, **caractérisé en ce que** la réaction en phase gazeuse catalysée par voie hétérogène est une oxydation partielle catalysée par voie hétérogène d'un composé organique.

29. Procédé selon la revendication 28, **caractérisé en ce que** l'oxydation partielle catalysée par voie hétérogène est l'oxydation partielle de propène en acroléine et/ou en acide acrylique.

30. Procédé selon la revendication 28, **caractérisé en ce que** l'oxydation partielle catalysée par voie hétérogène est l'oxydation partielle de propane en acide acrylique.

31. Procédé selon la revendication 28, **caractérisé en ce que** l'oxydation partielle catalysée par voie hétérogène est l'oxydation partielle de méthacroléine en acide méthacrylique.

32. Procédé selon la revendication 28, **caractérisé en ce que** l'oxydation partielle catalysée par voie hétérogène est l'oxydation partielle d'un hydrocarbure présentant au moins 4 atomes de carbone en anhydride de l'acide maléique.

33. Procédé selon la revendication 28, **caractérisé en ce que** l'oxydation partielle catalysée par voie hétérogène est l'oxydation partielle d'iso-butène en méthacroléine.
